Europäisches Patentamt

European Patent Office

Office européen des brevets

(19)

(11) Publication number: 0 369 566
A2

# (12) EUROPEAN PATENT APPLICATION

(21) Application number: 89302313.5

(22) Date of filing: 08.03.89

(51) Int. Cl.5: C12N 15/13, A61K 39/395

(30) Priority: 17.11.88 US 274105

(43) Date of publication of application:
23.05.90 Bulletin 90/21

(84) Designated Contracting States:
AT BE CH DE ES FR GB GR IT LI LU NL SE

(71) Applicant: HYBRITECH INCORPORATED
11095 Torreyana Road
San Diego California 92126(US)

(72) Inventor: Johnson, Mary Jacqueline
16231 El Carmino Real
Rancho Sante Fe California 90267(US)
Inventor: Phelps, Julie Lefevre
11264 Dalby Place
San Diego California 92126(US)

(74) Representative: Hudson, Christopher Mark et
al
Erl Wood Manor
Windlesham Surrey GU20 6PH(GB)

(54) Bifunctional chimeric antibodies.

(57) The present invention discloses novel biologically produced bifunctional chimeric antibodies, directed against human carcinoembryonic antigen and metal chelates, having two sets of antigen-specific variable regions of defined amino acid sequences. DNA constructs for the light and heavy chain variable regions comprising the novel antibodies of the invention are disclosed. Eukaryotic host cells capable of biological expression of the bifunctional chimeric antibodies and comprising the novel bifunctional chimeric antibody-encoding DNA constructs are also provided.

EP 0 369 566 A2

## BIFUNCTIONAL CHIMERIC ANTIBODIES

The present invention relates to monoclonal bifunctional antibodies directed against human carcinoembryonic antigen and metal chelates. More particularly, it relates to novel bifunctional chimeric monoclonal antibodies directed against these antigens, and DNA constructs encoding for such antibodies, for in vitro and in vivo application.

Monoclonal antibodies are becoming increasingly important for both in vitro application in immunoassays and for the in vivo diagnosis and treatment of disease. Monoclonal antibodies which are directed against human carcinoembryonic antigen ("CEA") are particularly useful for the in vivo imaging and treatment of tumors associated with certain carcinomas, including colorectal and breast carcinomas. Depending upon the clinical application, these monoclonal antibodies are generally conjugated with radionuclides, drugs or toxins.

Most available monoclonal antibodies, however, are derived from murine, i.e., mouse, hybridomas. The in vitro application of murine antibodies in immunoassays presents potential problems associated with false positive results which are attributable to the reaction of serum components with murine immunoglobulins. More importantly though, the in vivo application of murine antibodies in human medicine is often limited due to their inherent immunogenicity. The administration of murine antibodies will, in many patients, induce an immune response which results in a gradual decline in the efficacy of the antibodies during multiple dose regimens. This decrease in efficacy is attributable, at least in part, to the rapid clearance from circulation or alteration of pharmacokinetic properties of murine antibodies by the patient's immune response. The immunogenicity associated with murine monoclonal antibodies, therefore, precludes multiple dose administrations over an extended period of time and substantially impacts their potential clinical value. It has been suggested that the use of human monoclonal antibodies for clinical application would overcome the limitations associated with the use of murine monoclonal antibodies. However, human monoclonal antibodies having the desired specificity and affinity for tumor-associated antigens, such as human carcinoembryonic antigen, are technically difficult to prepare.

Chimeric antibodies, in which the binding or variable regions of antibodies derived from one species are combined with the constant regions of antibodies derived from a different species, have been constructed by recombinant DNA methodology. Chimeric antibodies are described, for example, in European Patent Publication 173494; Shaw, et al., J. Immun., 138:4534 (1987), Sun, L.K. et al., Proc. Natl. Acad. Sci. USA, 84:214-218 (1987); Neuberger, M.S. et al., Nature, 314:268 (1985), Boulianne, G.L. et al., Nature, 312:643-646 (1984); and Morrison, S.L. et al., Proc. Natl. Acad. Sci. USA, 81:6851-6855 (1984). Typically, the variable region of a murine antibody is joined with the constant region of a human antibody. It is expected that as such chimeric antibodies are largely human in composition, they will be substantially less immunogenic than murine antibodies. Accordingly, chimeric monoclonal antibodies are highly desirable for in vivo application.

In addition to the importance of constructing chimeric antibodies, it is also desirable to construct bifunctional antibodies. Bifunctional antibodies are those antibodies which comprise a light chain and a heavy chain from one antibody which recognizes a first specific antigen, in association with a light chain and a heavy chain from a second antibody which recognizes a second specific antigen. Such antibodies with dual specificities are quite useful in the prognosis, diagnosis and treatment of disease states. For example, if one set of light and heavy chains of a bifunctional antibody recognize a tumor associated antigen, while the corresponding light and heavy chains recognize a metal chelate, the bifunctional antibody can be used for tumor imaging and therapy.

Upon introduction of the bifunctional antibody into the system of a patient, the light and heavy chains of one part of the antibody will specifically bind to the tumor associated antigen. Introduction of a metal chelate into the patient's system will provide a known quantity of antigen with which the metal chelate-binding arms of the bifunctional antibody can react. The free metal chelates, being very small molecules, will quickly pass from the patients system, thereby allowing enhanced imaging of any tumor site where the bifunctional antibodies have bound.

The present invention specifically provides chimeric bifunctional antibodies wherein one light and heavy chain recognize CEA, while the second light and heavy chain recognize metal chelates. The specific metal chelate-binding light and heavy chain variable regions of the present bifunctional chimeric antibody were derived from monoclonal antibody CHA255, which was disclosed in Reardon et al., (1985) Nature 316:265-268. The CHA255 antibody most efficiently binds to the EDTA-chelate of Indium (III), but also recognizes the EDTA chelates of Iron (II) and Cadmium (II).

While the general concepts of bifunctional and chimeric antibodies have been described, there exists a need for the development of novel bifunctional chimeric antibodies having specificity for predetermined antigens of interest, particularly human carcinoembryonic antigen and metal chelates, and variable regions of defined amino acid sequences. Further, there exists a need for the development of DNA constructs having defined DNA coding sequences for light and heavy chain variable regions which comprise novel bifunctional chimeric antibodies, and which are capable of expressing these novel bifunctional chimeric proteins in eukaryotic cells. The present invention meets these needs.

For purposes of the present invention, as disclosed and claimed herein, the following terms are as defined below.

A - deoxyadenosine.

Ala - an alanine residue.

$Ap^R$ - the ampicillin-resistant phenotype or gene conferring same.

Arg - an arginine residue.

Asn - an asparagine residue.

Asp - an aspartic acid residue.

Bifunctional antibody - an antibody comprising a light chain variable region and a heavy chain variable region which specifically react with a first antigen in conjunction with a light chain variable region and a heavy chain variable region which specifically react with a second and different antigen.

C - deoxycytosine.

Chimeric antibody - an antibody comprising a variable region from one species, typically mouse, joined to a constant region from a second and different species, typically human.

Cys - a cysteine residue.

G - deoxyguanosine.

Gln - a glutamine residue.

Glu - a glutamic acid residue.

Gly - a glycine residue.

$G418^R$ - the G418-resistant phenotype or gene conferring same. May also be identified as $Km^R$.

His - a histidine residue.

Ile - an isoleucine residue.

IVS - DNA encoding an intron, also called an intervening sequence.

Lys - a lysine residue.

Met - a methionine residue.

MoAB - monoclonal antibody.

Nascent protein - the polypeptide produced upon translation of a mRNA transcript, prior to any post-translational modifications.

pA - a DNA sequence encoding a polyadenylation signal.

Phe - a phenylalanine residue.

Pro - a proline residue.

Promoter - a DNA sequence that directs transcription of DNA into RNA.

Recombinant DNA Cloning Vector - any autonomously replicating agent, including, but not limited to, plasmids and phages, comprising a DNA molecule to which one or more additional DNA segments can be or have been added.

Recombinant DNA Expression Vector - any recombinant DNA cloning vector into which a promoter has been incorporated.

Replicon - A DNA sequence that controls and allows for autonomous replication of a plasmid or other vector.

Restriction Fragment - any linear DNA sequence generated by the action of one or more restriction endonuclease enzymes.

Sensitive Host Cell - a host cell that cannot grow in the presence of a given antibiotic or other toxic compound without a DNA segment that confers resistance thereto.

Ser - a serine residue.

Structural Gene - any DNA sequence that encodes a functional polypeptide, inclusive of translational start and stop signals.

T - deoxythymidine.

$Tc^R$ - the tetracycline-resistant phenotype or gene conferring same.

Thr - a threonine residue.

Trp - a tryptophane residue.

Tyr - a tyrosine residue.

Val - a valine residue.

Figure 1 - Restriction Site and Function Maps of Plasmids pMLCE-10 and pHFK-1. For the purpose of this disclosure, the Figures are not drawn exactly to scale.

Figure 2 - Restriction Site and Function Maps of Plasmids pHKCE-10 and pGCEMK.

Figure 3 - Restriction Site and Function Maps of Plasmids pMHCE-30 and pHG1Z.

Figure 4 - Restriction Site and Function Maps of Plasmids pHGCEM-30 and pNCEMG1.

Figure 5 - Restriction Site and Function Map of Plasmid pNCEMKG1.

Figure 6 - Restriction Site and Function Maps of Plasmids pMLCH1 and pMLCH1dB.

Figure 7 - Restriction Site and Function Map of Plasmid pGCHAK.

Figure 8 - Restriction Site and Function Map of Plasmids pUCVHInc-1A and pHG1-CHA.

Figure 9 - Restriction Site and Function Map of Plasmid pGCHAG1.

Figure 10 - Restriction Site and Function Map of Plasmid pGCHAKG1.

The present invention is a bifunctional chimeric monoclonal antibody (and DNA compounds encoding said antibody) wherein one light chain variable region and one corresponding heavy chain variable region comprise light and heavy chain variable regions which specifically recognize human carcinoembryonic antigen and the second light chain variable region and corresponding second heavy chain variable region comprise light and heavy chain variable regions which specifically recognize metal chelates and the constant regions of all light and heavy chains comprise human constant regions. The bifunctional chimeric monoclonal antibody light chain variable region which recognizes human carsinoembryonic antigen has an amino acid sequence substantially the same as:

Asp - Ile - Val - Met - Thr - Gln - Ser - Gln - Lys Phe - Met - Ser - Thr - Ser - Val - Gly - Asp - Arg Val - Ser - Ile - Thr - Cys - Lys - Ala - Ser - Gln Asn - Val - Arg - Thr - Ala - Val - Ala - Trp - Tyr Gln - Gln - Lys - Pro - Gly - Gln - Ser - Pro - Lys Ala - Leu - Ile - Tyr - Leu - Ala - Ser - Asn - Arg Tyr - Thr - Gly - Val - Pro - Asp - Arg - Phe - Thr Gly - Ser - Gly - Ser - Gly - Thr - Asp - Phe - Thr Leu - Thr - Ile - Thr - Asn - Val - Gln - Ser - Glu Asp - Leu - Ala - Asp - Tyr - Phe - Cys - Leu - Gln His - Trp - Asn - Tyr - Pro - Leu - Thr - Phe - Gly Ala - Gly - Thr - Lys - Leu - Glu- Leu - Lys - Arg.

The bifunctional chimeric monoclonal antibody heavy chain variable region which recognizes human carcinoembryonic antigen has an amino acid sequence substantially the same as:

Asp - Val - Gln - Leu - Val - Glu - Ser - Gly - Gly Gly - Leu - Val - Gln - Pro - Gly - Gly - Ser - Arg Lys - Leu - Ser - Cys - Ala - Ala - Ser - Gly - Phe Thr - Phe - Ser - Asn - Phe - Gly - Met - His - Trp Ile - Arg - Gln - Als - Pro - Glu - Lys - Gly - Leu Gly - Trp - Val - Ala - Tyr - Ile - Ser - Gly - Gly Ser - Ser - Thr - Ile - Tyr - Tyr - Ala - Asp - Thr Val - Lys - Gly - Arg - Phe - Thr - Ile - Ser - Arg Asp - Asn - Pro - Arg - Asn - Thr - Leu - Phe - Leu Gln - Met - Thr - Ser - Leu - Arg - Ser - Glu - Asp Thr - Ala - Met - Phe - Tyr - Cys - Ala - Arg - Asp Tyr - Tyr - Ala - Asn - Asn - Tyr - Trp - Tyr - Phe Asp - Val - Trp - Gly - Ala - Gly - Thr - Thr - Val Thr - Val - Ser - Ala.

The bifunctional chimeric monoclonal antibody light chain variable region which recognizes metal chelates has an amino acid sequence substantially the same as:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly.

The bifunctional chimeric monoclonal antibody heavy chain variable region which recognizes metal chelates has an amino acid sequence substantially the same as:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala.

The compounds of the present invention represent recombinant bifunctional chimeric monoclonal antibody CEM/CHA. Due to the complementary nature of DNA base pairing, the sequence of one strand of a double-stranded DNA molecule is sufficient to determine the sequence of the opposing strand. The nucleotide sequence of the gene encoding the bifunctional chimeric monoclonal antibody light chain variable region which recognizes human carcinoembryonic antigen is:

GAC - ATT - GTG - ATG - ACC - CAG - TCT - CAA - AAA TTC - ATG - TCC - ACA - TCA - GTA - GGA - GAC - AGG GTC - AGC - ATC - ACC - TGC - AAG - GCC - AGT - CAG AAT - GTT - CGT - ACT - GCT - GTT - GCC - TGG - TAT CAA - CAG - AAA - CCA - GGG - CAG - TCT - CCT - AAA GCA - CTG - ATT - TAC - TTG - GCA - TCC - AAC - CGG TAC - ACT - GGA - GTC - CCT - GAT - CGC - TTC - ACA GGC - AGT - GGA - TCT - GGG - ACA - GAT - TTC - ACT CTC - ACC - ATT - ACC - AAT - GTG - CAA - TCT - GAA GAC - CTG - GCA - GAT - TAT - TTC - TGT - CTG - CAA CAT - TGG - AAT - TAT - CCG - CTC - ACG - TTC - GGT GCT - GGG - ACC - AAG - CTG - GAG - CTG - AAA - CGG.

The nucleotide sequence of the gene encoding the bifunctional chimeric monoclonal antibody heavy chain variable region which recognizes human carcinoembryonic antigen is:

GAT - GTG - CAG - CTG - GTG - GAG - TCT - GGG - GGA GGC - TTA - GTG - CAG - CCT - GGA - GGG - TTC - CGG AAA - CTC - TCC - TGT - GCA - GCC - TCT - GGA - TTC ACT - TTC - AGT - AAC - TTT - GGA - ATG - CAC - TGG ATT - CGT - CAG - GCT - CCA - GAG - AAG - GGA - CTG GAG - TGG - GTC - GCA - TAC - ATT - AGT - GGT - GGC AGT - AGT - ACC - ATC - TAC - TAT - GCA - GAC - ACA GTG - AAG - GGC - CGA - TTC - ACC - ATC - TCC - AGA GAC - AAT - CCC - AGG - AAC - ACC - CTC - TTC - CTG CAA - ATG - ACC - AGT- CTA - AGG - TCT - GAG - GAC ACG - GCC - ATG - TTT - TAC - TGT - GCA - AGA - GAT TAC - TAC - GCT - AAC - AAC - TAC - TGG - TAC - TTC GAT - GTC - TGG - GGC - GCA - GGG - ACC - ACG - GTC ACC - GTC - TCC - TCA - GCC.

The nucleotide sequence of the gene encoding the bifunctional chimeric monoclonal antibody light chain variable region which recognizes metal chelates is:

CAG GCT GTT GTG ACT CAG GAA TCT GCA CTC ACC ACA TCA CCT GGT GAA ACA GTC ACA CTC ACT TGT CGC TCA AGT ACT GGG GCT GTT ACA ACT AGT AAC TAT GCC AAC TGG GTC CAA GAA AAA CCA GAT CAT TTA TTC ACT GGT CTA ATA GGT GGT ACC AAT AAC CGG GCT CCA GGT GTT CCT GCC AGA TTC TCA GGC TCC CTG ATT GGA GAC AAG GCT GCC CTC ACC ATC ACA GGG GCA CAG ACT GAA GAT GAG GCA AGA TAT TTC TGT GCT CTA TGG TAC AGC AAC CTC TGG GTA TTC GGT GGA GGA ACC AAA CTG ACT GTC CTA GGG

The nucleotide sequence of the gene encoding the bifunctional chimeric monoclonal antibody heavy chain variable region which recognizes metal chelates is:

GAA GTG ACG CTG GTG GAG TCT GGG GGA GAC TCA GTG AAG CCT GGA GGG TCC CTG AAA CTC TCC TGT GCA GCC TCT GGA TTC ACT TTA AGT GGT GAA ACC ATG TCT TGG GTT CGC CAG ACT CCG GAG AAG AGG CTG GAG TGG GTC GCA ACC ACT CTT AGT GGT GGT GGT TTC ACC TTC TAT TCA GCC AGT GTG AAG GGT CGT TTC ACC ATC TCC AGA GAC AAT GCC CAG AAC AAC CTC TAT CTA CAA CTG AAT AGT CTG AGG TCT GAG GAC ACG GCC TTG TAT TTC TGT GCA AGT CAT CGG TTT GTT CAC TGG GGC CAC GGG ACT CTG GTC ACT GTC TCT GCA GCC.

The novel DNA compounds of the present invention are derived from genomic DNA clones prepared from two separate hybridoma cell lines. The light and heavy chain variable region genes which express immunoglobulin chains which recognize human carcinoembryonic antigen, were cloned from a genomic DNA library of hybridoma CEM 231.6.7. Murine hybridoma CEM 231.6.7 is part of the permanent stock culture collection of the American Type Culture Collection, Rockville, MD, and is available to the public under the accession number ATCC HB 9620. The light and heavy chain variable region genes which express immunoglobulin chains which recognize metal chelates, were cloned from a genomic DNA library of hybridoma CHA255.5. Murine hybridoma CHA255.5 is disclosed in Reardon et al., (1985) Nature 316:265-268. Murine hybridoma CHA255.5 is also disclosed in Meares and David, U.S. Patent No. 4,722,892, issued February 2, 1988, the entire disclosure of which is herein incorporated by reference. The genes which encode the human constant regions of all light and heavy chain constructions were cloned from a gene library derived from human blood cells. The present invention provides a needed benefit to medical science in that, for the first time, large amounts of bifunctional chimeric antibodies can now be biologically produced.

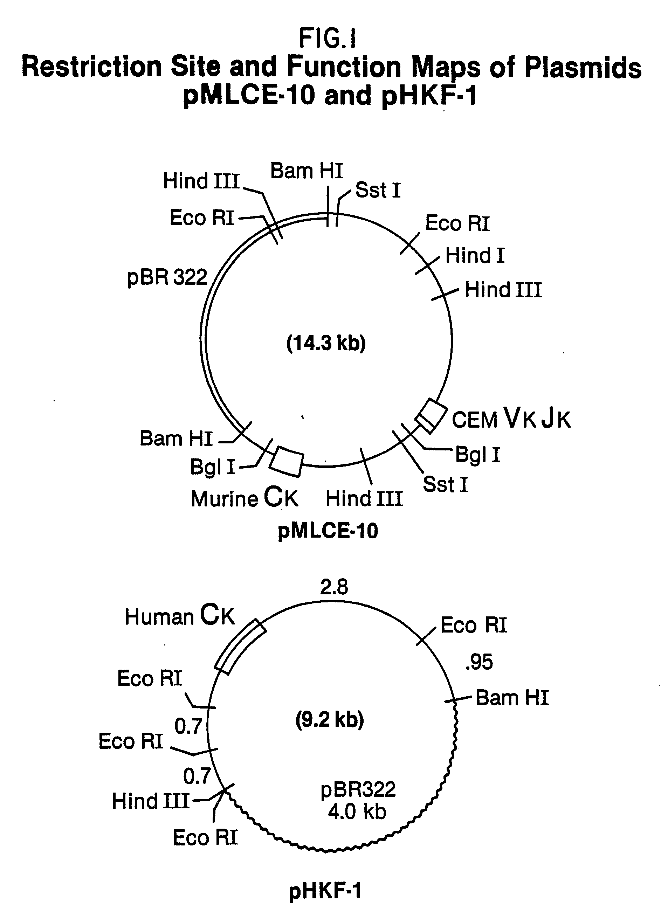

Plasmid pMLCE-10 comprises the genomic sequence of the light chain variable region of monoclonal antibody CEM, which recognizes human carcinoembryonic antigen. Plasmid pMLCE-10 is part of the permanent collection of the American Type Culture Collection (deposited March 1, 1988) and is available under accession number ATCC 67639. Plasmid pHKF-1 comprises the genomic sequence of the light chain constant region of human antibody. Plasmid pHKF-1 is part of the ATCC permanent collection (deposited March 1, 1988) under accession number ATCC 67637. Restriction site and function map of plasmids pMLCE-10 ad pHKF-1 are presented in Figure 1 of the accompanying drawings.

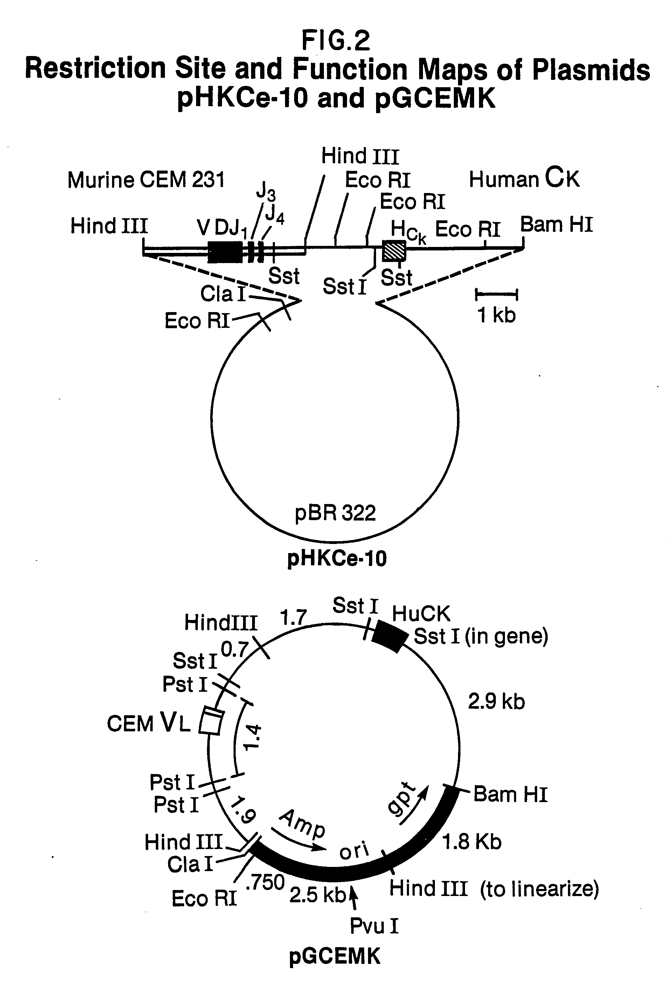

Plasmid pHKCE-10 was constructed by isolating the approximately 3.8 kb HindIII fragment containing the CEM light chain variable region gene from plasmid pMLCE-10 and ligating this fragment into HindIII digested plasmid pHKF-1. Plasmid pSV2gpt (available from the ATCC under accession number ATCC 37145) was digested with restriction enzyme EcoRI and ClaI linkers (sequence dCATCCGATG) were ligated into the EcoRI site to form plasmid pSV2gpt Cla. Plasmid pHKCE-10 was next digested with restriction enzymes ClaI and BamHI and the approximately 9.0 kb ClaI/BamHI restriction fragment, which comprises the CEM light chain variable region gene linked to the human light chain constant region gene, was isolated. Plasmid pSV2gpt-Cla was also digested with restriction enzymes ClaI and BamHI and the approximately 4.5 kb ClaI/BamHI restriction fragment was isolated. The about 9.0 kb fragment from plasmid pHKCE-10 was ligated into the about 4.5 kb vector fragment of plasmid pSV2gpt-Cla to form expression plasmid pGCEMK. Restriction site and function maps of plasmids pHKCE-10 and pGCEMK are presented in Figure 2 of the accompanying drawings.

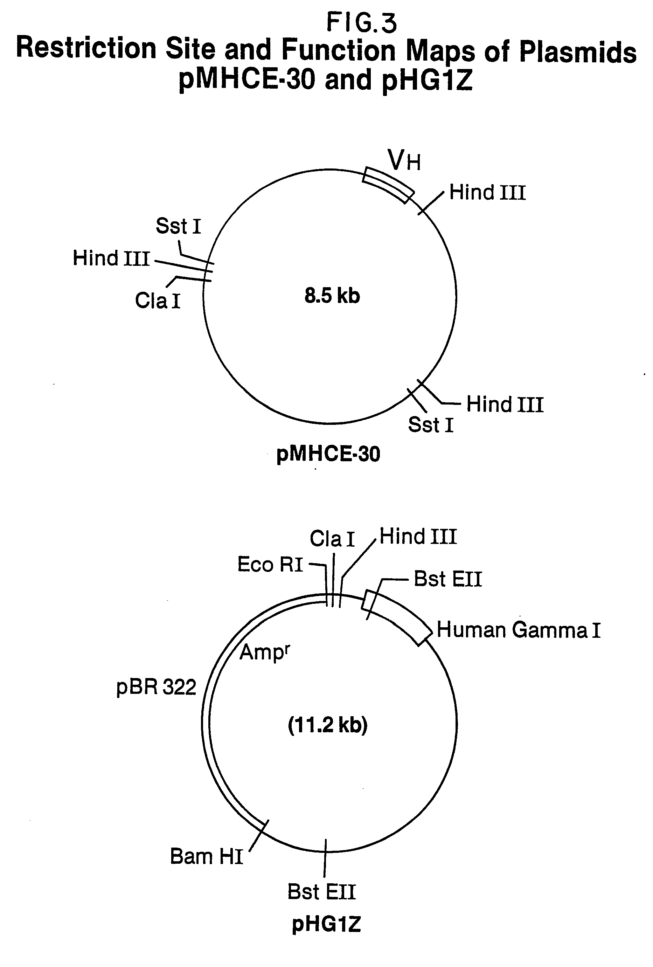

Plasmid pMHCE-30 comprises the genomic sequence of the heavy chain variable region of monoclonal antibody CEM, which recognizes human carcinoembryonic antigen. Plasmid pMHCE-30 is part of the ATCC collection (deposited March 1, 1988) and is available under accession number ATCC 67640. Plasmid pHG1Z comprises the genomic sequence of the heavy chain variable region of human antibody. Plasmid pHG1Z is deposited with the ATCC (deposited March 1, 1988) under accession number ATCC 67638. Restriction site and function maps of plasmid pMHCE-30 and pHG1Z are presented in Figure 3 of the accompanying drawings.

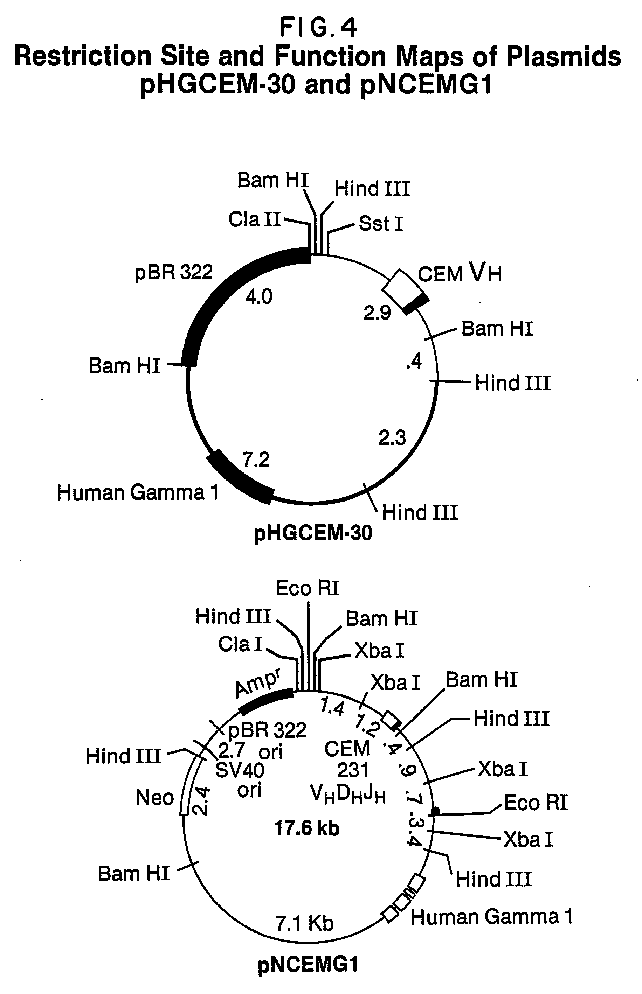

Plasmid pHGCEM-30 was constructed by isolating the approximately 5.3 kb ClaI/HindIII fragment containing the CEM heavy chain variable region gene from plasmid pMHCE-30 and ligating this fragment into ClaI/HindIII digested vector pHG1Z. Inasmuch as plasmid pMHCE-30 contains more than one BamHI site, the 5.3 kb ClaI/HindIII restriction fragment of plasmid pMHCE-30 is most easily isolated following a total ClaI digested and a subsequent partial BamHI digestion. Plasmid pSV2neo (ATCC 37149) was digested with restriction enzyme EcoRI and ClaI linkers (dCATCCGATG) were ligated into the EcoRI site to form plasmid pSV2neo-Cla. Plasmid pSV2neo-Cla was then totally digested with restriction enzymes BamHI and ClaI and the about 4.5 kb vector fragment was isolated. Plasmid pHGCEM-30 was totally digested with restriction enzyme ClaI, then partially digested with restriction enzyme BamHI and the about 12.7 kb restriction fragment, comprising the gene encoding the CEM heavy chain variable region linked to the gene encoding the human heavy chain constant region, was isolated. The about 12.7 kb ClaI/BamHI restriction fragment of plasmid pHGCEM-30 was ligated into the about 4.5 kb ClaI/BamHI vector fragment of plasmid pSV2-neo-Cla to form expression vector pNCEMG1. Restriction site and function maps of plasmids pHGCEM-30 and pNCEMG1 are presented in Figure 4 of the accompanying drawings.

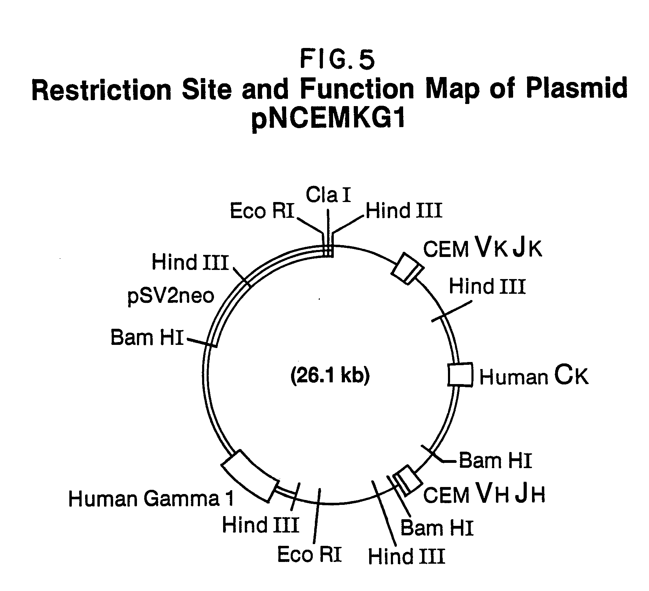

Plasmid PGCEMK was digested with restriction enzymes ClaI and BamHI and the approximately 8.9 kb ClaI/BamHI restriction fragment was isolated. Plasmid pNCEMG1 was also digested with restriction enzymes ClaI and BamHI and the approximately 17.6 kb ClaI/BamHI restriction fragment was isolated. The about 8.9 kb ClaI/BamHI restriction fragment of plasmid pGCEMK was then ligated into the about 17.6 kb ClaI/BamHI restriction fragment of plasmid pNCEMG1 to form expression vector pNCEMKG1. Plasmid pNCEMKG1 comprises the gene encoding the CEM light chain variable region linked to the gene encoding the human light chain constant region as well as the gene encoding the CEM heavy chain variable region linked to the gene encoding the human heavy chain variable region. Plasmid pNCEMKG1 also contains the neomycin resistance-conferring gene. A restriction site and function map of plasmid pNCEMKG1 is presented in Figure 5 of the accompanying drawings.

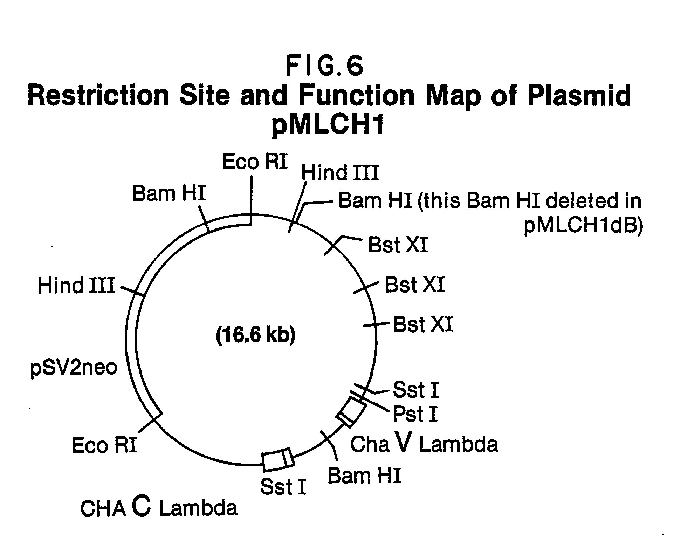

Plasmid pMLCH1 comprises the genomic sequence encoding the light chain variable region of monoclonal antibody CHA, which recognizes metal chelates. Plasmid pMLCH1 can be conventionally isolated from E. coli K12 HB101/pMLCH1, a strain deposited and made part of the permanent stock culture collection of the Northern Regional Research Laboratory (NRRL), Peoria, Illinois. A culture of E. coli K12 HB101/pMLCH1 (deposited November 14, 1988) can be obtained from the NRRL under the accession number NRRL B-18432. Plasmid pMLCH1 was digested with restriction enzyme BamHI, then treated with Klenow enzyme to remove the BamHI site which occurs 5' to the structural gene in the plasmid. Religation of this vector fragment created plasmid pMLCH1dB. Restriction site and function maps of plasmids pMLCH1 and pMLCH1dB are presented in Figure 6 of the accompanying drawings.

Plasmid pMLCH1dB was digested with restriction enzymes HindIII and BamHI and the approximately 5.75 kb HindIII/BamHI restriction fragment, which comprises the gene encoding the CHA heavy chain variable region, was isolated. Plasmid pBR322 was also digested with restriction enzymes HindIII and BamHI and the large vector fragment was purified. The about 5.75 kb HindIII/BamHI restriction fragment of plasmid pMLCH1dB was then ligated into the HindIII/BamHI-digested vector fragment of plasmid pBR322 to form plasmid pMLCH2. Plasmid pMLCH2 was then digested with restriction enzymes ClaI and BamHI and the approximately 5.75 kb ClaI/BamHI restriction fragment was isolated and ligated into ClaI/BamHI digested plasmid pSV2gpt-Cla to form plasmid pGCHA.

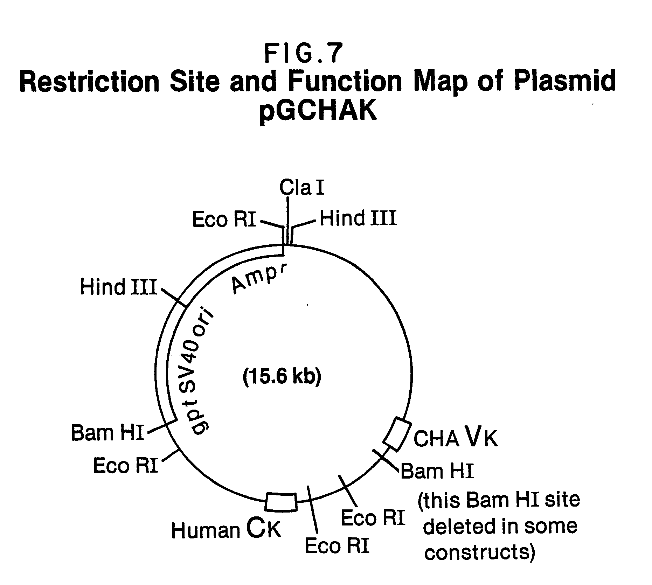

Plasmid pGCHA was digested with restriction enzyme BamHI and the 11.2 kb fragment was purified. Plasmid pHKF1, which comprises the gene encoding the human light chain constant region, was digested with restriction enzyme HindIII, filled in with Klenow, phosphorylated BamHI (NEB) linkers were added, then the vector was cut with BamHI, and the approximately 5.2 kb BamHI restriction fragment was isolated. The about 5.2 kb BamHI restriction fragment of plasmid pHKF1 was then ligated into the BamHI-digested vector fragment of plasmid pGCHA to form expression plasmid pGCHAK, which comprises the gene encoding the CHA light chain variable region linked to the gene encoding the human light chain constant region. A restriction site and function map of plasmid pGCHAK is presented in Figure 7 of the accompanying drawings.

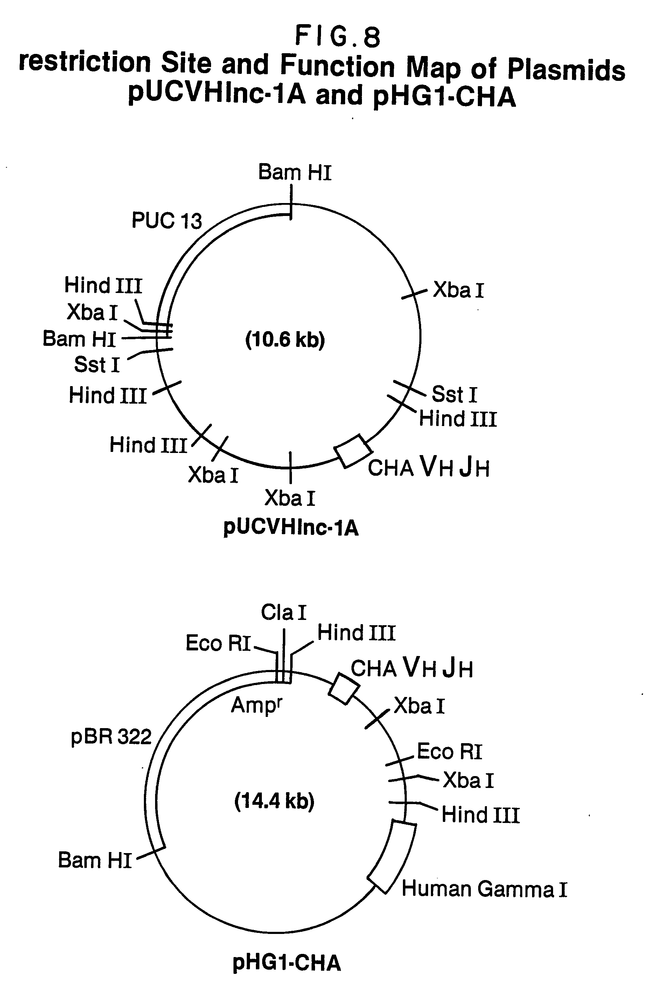

Plasmid pUCVHlnc-1A comprises the genomic sequence encoding the heavy chain variable region of monoclonal antibody CHA, which recognizes metal chelates. Plasmid pUCVHlnc-1A can be conventionally isolated from E. coli K12 HB101/pUCVHlnc-1A, a strain deposited (November 14, 1988) and made part of the permanent stock culture collection of the NRRL. A culture of E. coli K12 HB101/pUCVHlnc-1A can be obtained under the accession number NRRL B-18433. Plasmid pUCVHlnc-1A was digested with restriction enzyme HindIII and the approximately 3.4 kb HindIII restriction fragment, which comprises the gene encoding the CHA heavy chain variable region, was isolated. Plasmid pHG1Z (ATCC 67638) which comprises the gene encoding the human heavy chain constant region, was also digested with restriction enzyme HindIII and then treated with Calf Intestine Alkaline Phosphatase to create blunt ends. The about 3.4 kb HindIII restriction fragment of plasmid pUCVHlnc-1A was ligated into the HindIII-digested, phosphatased vector fragment of plasmid pHG1Z to form plasmid pHG1-CHA. Restriction site and function maps of plasmids pUCVHlnc-1A and pHG1-CHA are presented in Figure 8 of the accompanying drawings.

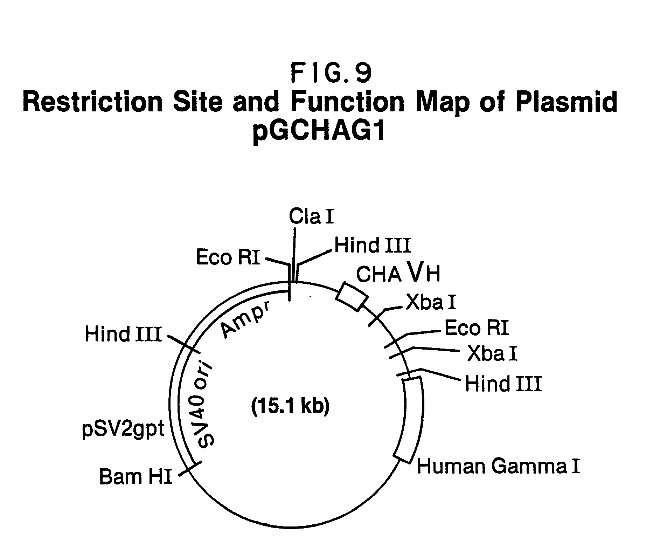

Plasmid pHG1-CHA, which comprises the gene encoding the CHA heavy chain variable region linked to the gene encoding the human heavy chain constant region, was digested with restriction enzymes ClaI and BamHI and the approximately 10.5 kb ClaI/BamHI restriction fragment was isolated. Plasmid pSV2neo-Cla was next digested with restriction enzymes BamHI and ClaI and the approximately 5.0 kb ClaI/BamHI vector fragment was purified. The about 10.5 kb ClaI/BamHI restriction fragment of plasmid pHG1-CHA was ligated into the about 5.0 kb ClaI/BamHI vector fragment of plasmid pSV2gpt-Cla to form expression vector pGCHAG1. A restriction site and function map of plasmid pGCHAG1 is presented in Figure 9 of the accompanying drawings.

Plasmid pGCHAG1 was digested with restriction enzyme ClaI and the approximately 15.05 kb ClaI restriction fragment was isolated. Plasmid pGCHAK was digested with restriction enzyme BamHI and ClaI linkers

$$5'-GATCCGGGATCGATGGGG-3'$$
$$| | | | | | | | | | | | | | |$$
$$3'-GCCCTAGCTACCCCCTAG-5'$$

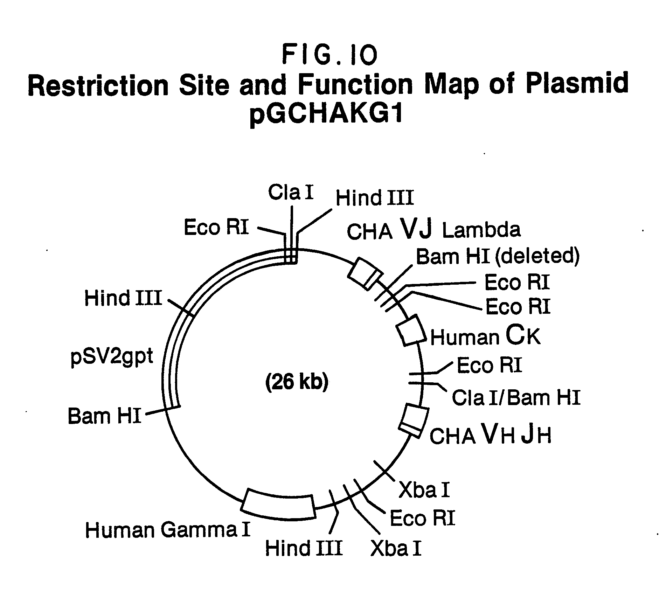

were ligated into the BamHI site to form plasmid pGCHAK-Cla. Plasmid pGCHAK-Cla was then digested with restriction enzyme ClaI and the approximately 10.85 kb ClaI restriction fragment was isolated. The about 10.85 kb ClaI restriction fragment of plasmid pGCHAK-Cla was then ligated into the about 15.05 kb ClaI fragment of vector pGCHAG1 to form expression plasmid pGCHAKG1. Plasmid pGCHAKG1 comprises the gene encoding the CHA light chain variable region joined to the gene encoding the human light chain constant region as well as the gene encoding the CHA heavy chain variable region joined to the gene encoding the human heavy chain constant region. Plasmid pGCHAKG1 also comprises the gene which confers resistance to the mycophenolic acid. A restriction site and function maps of plasmid pGCHAKG1 is presented in Figure 10 of the accompanying drawings.

The present DNA compounds which encode recombinant CEM and CHA immunoglobulins and derivatives are especially preferred for the construction of vectors for transformation and expression of the various antibody chains in mammalian and other eukaryotic cells. Many mammalian host cells possess the necessary cellular machinery for the recognition and proper processing of the signal peptides present on the amino-terminus of the various antibody chains embodied in the present invention. Some mammalian host cells also provide the post-translational modifications, such as glycosylation, that are observed in antibody molecules. A wide variety of vectors exist for the transformation of eukaryotic host cells, and the specific vectors exemplified below are in no way intended to limit the scope of the present invention.

The various expression vectors of the present invention can be transformed into and expressed in a variety of eukaryotic, especially mammalian, host cells. The expression vectors also comprise sequences that allow for replication in E. coli. Expression of antibodies occurs in host cells in which the particular promoter associated with the antibody's structural gene functions. Skilled artisans will understand that a variety of eukaryotic host cells can be used to express the various antibody chains of the present invention. The SP2/0-Ag14 cell line is a myeloma cell line which ordinarily does not secrete antibody. Following transfection of cell line SP2/0 with plasmids pGCHAKG1 and pNCEMKG1, the transfected cell line secretes bifunctional chimeric CEM/CHA antibody into the culture fluid. Subcloning experiments, followed by conversion of the secreting cells into serum-free media, demonstrated that the bifunctional antibodies could be expressed at levels up to 15 μg/ml/$10^6$ cells. While SP2/0 cells are the preferred host cells for the expression vectors of the present invention, skilled artisans recognize that a wide variety of cells may be utilized to express the bifunctional chimeric antibodies or derivatives of the present invention.

The host cells used in the invention may be transformed in a variety of ways by standard transfection procedures well known in the art. Among the standard transfection procedures which may be used are electroporation techniques, protoplast fusion and calcium-phosphate precipitation techniques. Such techniques are generally described by Toneguzzo, F. et al., Mol. and Cell. Biol., 6:703-706 (1986); Chu, G., et al., Nucleic Acid Res., 15:1311-1325 (1987); Rice, D., et al., Proc. Natl. Acad. Sci. USA, 79:7862-7865 (1979) and; Oi, V., et al, Proc. Natl. Acad. Sci. USA, 80:825-829 (1983).

Preferably, the recombinant expression vectors comprising the CEM and CHA constructs of the invention are transfected sequentially into host cells. For example, the expression vectors comprising the CHA constructs are first transfected into the host cells and transformed host cells expressing the CHA immunoglobulin chains are selected by standard procedures known in the art. See, e.g. Engvall, E. and Perlmann, P., Immunochemistry, 8:871-874 (1971) or Meares and David, U.S. Patent No. 4,722,892, issued February 2, 1988, the entire teaching of which is herein incorporated by reference. The expression vectors comprising the CEM immunoglobulin gene constructs are, thereafter, transfected into the selected host cells. However, it will be recognized that both the CHA and CEM constructs can be simultaneously introduced into the host cells or introduced in inverse order. For instance, it would be possible by methods discussed herein to construct a vector containing two light chains of different specifications or two heavy chains of different specificities and transfect sequentially or simultaneously those vectors into host cells. Alternatively, both the CEM and CHA gene constructs can be combined on a single expression vector, or the two DNA's could be linearized and ligated together prior to transformation into cells. Following transfection and selection, standard assays are performed for the detection of antibodies directed against CEA and metal chelates for the identification of transformed cells expressing the bifunctional chimeric antibodies of the present invention.

Higher levels of expression can be attained by removing the SV40 enhancer from the expression vectors formed from the pSV2-class vectors. Nearly all genomic immunoglobulin genes contain enhancer sequences. The murine variable region genes found on the plasmids of the present invention are all found in conjunction with murine enhancer sequences cloned along with the immunoglobulin genes from the genomic libraries. Skilled artisans will recognize that these enhancer sequences can be moved to many different sites on the expression vectors without changing the ability of transfected cells to produce these immunoglobulin chains. However, the removal of the SV40 enhancer from expression vectors pNCEMKG1 or pGCHAKG1 (thereby forming plasmids pGCHAKG1(E-) and pNCEMKG1(E-)) can lead to an increase in the levels of bifunctional chimeric CEM/CHA antibody from SP2/0 cells.

The CEM kappa promoter, found on plasmid pGCEMK is also useful to increase the levels of expression of heterologous immunoglobulin chains. When stated in this context, heterologous immunoglobulin chains are defined as any immunoglobulin chain other than the murine kappa light chain encoded on plasmid pGCEMK. Plasmid pGCHAK-2, which comprises the murine lambda CHA variable region and human constant region genes driven by the CEM kappa promoter on an SV40 enhancer-containing vector is constructed according to the teaching of Example 7. Plasmid pGCHAK-3, which comprises the murine lambda CHA variable region and human constant region genes driven by the CEM kappa promoter on an SV40 enhancerless vector is also constructed according to the teaching of Example 7. By replacing plasmid pGCHAK with plasmid pGCHAK-2 in the scheme for building plasmid pGCHAKG1, one can easily construct plasmid pGCHAKG1-2. In an analogous manner, plasmid pGCHAKG1-3 can be constructed by replacing plasmid pGCHAG1 with plasmid pGCHAGI(E-), and replacing pGCHAK with pGCHAK-2 in the scheme for the construction of plasmid pGCHAKG1. SP2/0 cells transfected with either of plasmids pGCHAKG1-2 or pGCHAKG1-3, then subsequently transfected with vector pNCEMKG1, will demonstrate higher levels of expression of bifunctional chimeric CEM/CHA antibody than do cells which are transfected with vectors pGCHAKG1 and pNCEMKG1.

After expression of the genes within the transfected host, the mature bifunctional chimeric CEM/CHA antibodies are secreted into the supernatant. As many recombinantly produced antibodies display unwanted heterogeneity (arising from an extraneous amino acid or amino acids appearing at the C-terminus of some gamma chains), the culture fluid is generally concentrated and treated with a solution of carboxypeptidase after culture collection. The bifunctional chimeric CEM/CHA antibodies can then be purified according to techniques well known in the art. (See Catherine B. Beidler, M.J. Johnson, and James R. Ludwig, Chimeric Antibodies Directed Against Human Carcinoembryonic Antigen, Attorney Docket No. H-7587A, U.S. Application No. 07/272,577, filed this even date, (European Patent Application No. 89302312.7) and M.J. Johnson, Chimeric Antibodies Directed Against Metal Chelates, Attorney Docket No. H-7589, U.S. Application No. 07/274,106, filed this even date, (European Patent Application No. 89302314.3) both of which are herein incorporated by reference.

The presence of bifunctional chimeric CEM/CHA antibodies can be detected in a wide variety of ways. One method is to coat polystyrene beads with monoclonal antibody CEV124, which recognizes CEA. Next, CEA is allowed to bind to the polystyrene/CEV124 complex. Alternatively, CEA can be allowed to bind directly to microtiter plate wells, rather than via an antibody linker. Supernatant containing bifunctional chimeric CEM/CHA antibodies is then incubated with the beads, thereby allowing the CEA-recognizing arms of CEM/CHA to bind to the CEA which is bound to the beads. Radiolabeled Indium-EDTA chelates are then added to the beads. The CHA arms of the bifunction chimeric CEM/CHA antibody will bind the Indium-EDTA chelates, thereby allowing for antibody titer determination using a gamma counter. Indium-EDTA chelates are disclosed in U.S. Patent No. 4,722,892, issued February 2, 1988, the disclosure of which is herein incorporated by reference. Skilled artisans recognize the virtually limitless methods which are available for the testing and assaying of the secreted CEM/CHA antibodies of the present invention.

The antibodies of the present invention, while having great utility for the in vitro detection of CEA in serum or tissue samples, are also quite useful for the detection of CEA in vivo. The chimeric portions of the antibody reduce the possibility of cross-reactivity and serum sickness, and therefore allow multiple dose regimens for both detection and treatment of disease states. Bifunotional chimeric CEM/CHA is infused and allowed to localize at the tumor site where CEA is located. The radionuclide Indium-EDTA chelate can be infused later, and inasmuch as the metal chelates are small molecules, the unbound metal chelates are rapidly cleared from the body, thereby reducing the possibility of damage to non-tumor tissue. If it is desired to image the tumor, preferably the radionuclide is selected on the basis of its emission of radiation, typically a gamma-photon, which can be detected by photoscanning techniques. If it is desired to treat the tumor to reduce its size, preferably the radionuclide emits an electron or an alpha-particle. Of the useful gamma-emitting isotopes, 111In is preferred. Among the useful isotopes emitting an alpha electron, Y90 is preferred.

In another application, the bifunctional chimeric CEM/CHA antibody is infused and allowed to localize at the site of the tumor associated CEA. A toxin or drug to which is conjugated the metal chelate can be subsequently infused to be bound by the antibody at the tumor site. In this application, if the metal is a radionuclide that, upon decay, changes atomic number to a metal whose chelate is not bound tightly to an antibody, the antibody will release the chelate and the associated drug or toxin in the vicinity of the tumorous cell to facilitate its entry into the cell. In such a case, the radionuclide is selected to have a half-life which insures that release does not occur prematurely or over too long a period.

The following examples are offered for purposes of illustration of the present invention and are not intended to limit it in any way. While the disclosed amino acid and nucleotide sequences herein disclosed comprise the constructed components of the chimeric bifunctional CEM/CHA antibody, it is understood that minor modifications to the sequences may result in variable regions which are substantially equivalent in the binding of CEA or metal chelates. These modifications are contemplated by the present invention provided the requisite specificities for CEA or metal chelates are retained.

## Examples

Murine hybridoma cells, designated as CEM 231.6.7, were used in the examples to derive and clone genomic DNA for variable light and heavy chain variable regions. Murine hybridoma CEM 231.6.7 was deposited with the American Type Culture Collection, Rockville, Maryland, under the accession number ATCC HB 9620. The cloned genomic DNA from murine hybridoma CEM 231.6.7, and human peripheral blood lymphocytes, were used for the construction of the chimeric genes.

Transfection of chimeric genes was accomplished by electroporation techniques, essentially as described by Toneguzzo, F., et al., Molecular and Cell. Biol., 6:703-706 (1986); and Chu, G. et al., Nucleic Acid Res., 15:1311-1325 (1987). The host cells SP2/0-Ag14 hybridoma cells were the recipients of the chimeric genes. The SP2/0-Ag14 hybridoma cells used are available from the American Type Culture Collection, Rockville, Maryland under the accession number ATCC CRL 1581.

## Example 1

## A. Isolation of DNA from CEM 231.6.7 Murine Hybridoma

Genomic DNA from CEM 231.6.7 was isolated by procedures essentially as described by Pellecer et al., Cell, 41:133 (1978). Approximately 1 x 10⁸ cells were collected by centrifugation (10′, 800 rpm IEC clinical centifuge). Cells were then washed 2x in Phosphate Buffered Saline (PBS). Cells were then resuspended in 4 ml of 10 mM Tris-HCl (pH 8.0) 2 mM EDTA, 40 mM NaCl (TEN), and lysed by the addition of 200 μl of 10% SDS and 42 μl of Protease K at 20 mg/ml (Sigma Chemicals, St. Louis, Missouri. The sample was incubated overnight at 37°C. DNA was extracted twice with an equal volume of phenol:chloroform:iso-amyl alcohol (25:24:1 ratio) and twice more with an equal volume of chloroform:iso-amyl alcohol (24:1 ratio) and dialyzed against 10 mM Tris-HCl (pH 8.0, 1 mM EDTA (TE Buffer) overnight. The DNA was then treated for 2 hours with RNAse A at 50 μg/ml (Sigma Chemicals, St. Louis, Missouri) followed by Protease K at 200 μg/ml for one hour at 37°C. DNA was extracted again as detailed above and dialyzed overnight. Following dialysis, DNA was ethanol precipitated by adding 1/10th volume of 2 M Na Acetate and 2 volumes of 95% ethanol. After 30 minutes at -20°C, the DNA was centrifuged at 8000 RPM for 30 minutes. The pellet was resuspended in TE buffer at a final concentration of 955 μg/ml.

## B. Construction of a Genomic Library for CEM231.6.7

Partial DNA digests of the CEM 231 genomic DNA were done by taking 10 μg of DNA in 11 μl TE buffer, 20 μl of H₂O, 15 μl of 10x Core Restriction Digest Buffer (500 mM pH8.0, 100 mM MgCl₂, 500 mM NaCl) and then aliquoted into tubes. Restriction enzyme Mbo I was added to each tube in units increasing from .0038 to .5 units/μl and placed at 37°C for one hour. Aliquots of the samples were then run on a 0.7% TBE (89 mM Tris 89 mM Borate 2 mM EDTA) agarose gel and electrophoresed overnight at 40 volts. From the photograph of this gel it was determined which digestion fractions contained DNA in the correct molecular weight range (12-24 kb). Two hundred μg of genomic DNA was then digested using the Mbo I units defined by the experiment described above (scaled up 20x) with an hour incubation at 37°C. This DNA was used to construct the EMBL-3 phage library using EMBL-3 phage DNA, commercially available from Stratagene, Inc. (La Jolla, CA), as described in the Stratagene protocol. The Stratagene protocol follows the techniques described in several laboratory manuals (e.g., Basic Methods in Molecular Biology, edited by L.G. Davis, M.D. Dibner and J.F. Battey, Elsevier, New York (1986). Following isolation on sucrose gradients, the large molecular weight CEM 231.6.7 DNA (12-24 kb) was ligated into the EMBL-3 phage arms using 100 ng of DNA, 100 ng of pre-isolated EMBL-3 phage arms, .5 μl of 10x Ligase Buffer (500 mM Tris-HCl pH8; 70 mM MgCl₂; 10 mM dithiothreitol (dtt) and T4 DNA ligase (2 units) at 4°C, overnight. Packaging was done using Stratagene's commercially available Gigapack-Gold In Vitro packaging system in accordance with their product protocols and using the host E. coli strain P2.392 supplied by Stratagene, Inc. 500 μl of Gigapack Gold packaging mix and 5 μl of the ligated phage were incubated at 22°C for 2 hours. 0.5 ml of phage dilution buffer (per liter 5.8 g NaCl, 2 g MgSO₄-6H₂O, 50 ml 1M Tris-HCl pH 7.5, 5 ml 2% gelatin) was added. Phage were then titered using standard protocols as described in Molecular Cloning, edited by T. Maniatis, E.F. Fritsch and J. Sambrook, Cold Spring Harbor Laboratories, Cold Spring Harbor, New York (1982). Following titration, the phage library was plated at a density of 20,000 plaques/100 mM plate using P2.392 cells and incubated at 37°C overnight.

## C. Identification and Isolation of Recombinant Phage ØMLCE-10 Including Murine CEM 231.6.7 v Kappa Gene

For the CEM 231.6.7 light (kappa) chain 4.8 x 10⁵ recombinant phage in E. coli P2.392, which is commercially available from Stratagene, Inc. (La Jolla, CA) were screened according to the protocol in Molecular Cloning, supra. This was done using murine kappa and murine J_L probes derived from plasmids obtained from Dr. Marc Shulman, University of Toronto, Toronto, Canada or synthetic probes whose sequence was derived from the General Bank Data Base (NIH Accession #J00545). The kappa oligonucleotide probe used was comprised of the sequence 5′-AGA-TGG-ATA-CAG-TTG-GTG-CAG-CAT-CAG-CCC-3′ and was synthesized by Molecular Biosystems, Inc. (San Diego, CA).

Duplicate filter lifts were made and phage hybridizing to both probes were analyzed by double southern blots as described in Molecular Cloning, supra, probed with the murine kappa and J_L probes which were radiolabeled with ³²P. Hybridizations were done as described in Molecular Cloning, supra. A single CEM 231.6.7 phage clone was selected on the basis of its hybridizing to both probes, indicating a light chain variable region gene rearrangement to a position directly contiguous to the C kappa gene region. This recombinant phage was designated ØMLCE-10.

D. Construction of Recombinant Plasmid pMLCE-10 Including CEM 231.6.7 Murine v Kappa Gene

ØMLCE-10 DNA was isolated and 20 µg were digested with BamHI (1 unit/µg) using React Buffer #3 from Gibco-BRL (Gaithersburg, Maryland) in a total of 220 µl. A 10 kb BamHI fragment was isolated by electrophoresis of the BamHI digested DNA in a .75% TBE agarose gel containing .5 µg/ml of ethidium bromide at 40 V overnight. Following visualization on a UV transparent light box the 10 kb fragment was electrophoresed onto DEAE 81 (Schleicher and Schuell, Keene, New Hampshire) paper followed by elution in 1 M NaCl and ethanol precipitation. The eluted fragment was then resuspended in 6 µl of TE buffer. The fragment was ligated to 50 ng of BamHI digested pBR322, available from the American Type Culture Collection (ATCC Designation 31344) by using 1 µl (50 ng) pf pBR322 DNA, 6 µl (300 ng) of BamHI 10 kb recombinant phage DNA ligase as described in Example 1B. E. coli HB101 competent cells, available from Gibco-BRL (Gaithersburg, Maryland), were transformed using standard procedures. HB101 cells were first thawed on ice; then 10 µl of the ligation reaction were mixed with 200 µl of cells and incubated on ice for 30 minutes. Following this, cells were heat shocked for 90 seconds at 42°C, then returned to ice for 2 minutes. One ml of LB broth (per liter 10 g NaCl, 5 g yeast extrct 10 g tryptone) was added and the cells incubated in a New Brunswick air shaker for one hour at 225 rpm, 37°C. Two hundred µl were then plated on LB-agarose with ampicillin (50 µg/ml) plated and incubated overnight at 37°C. Ampicillin resistant colonies were screened using colony screening methods as detailed in Molecular Cloning, supra, and Grunstein, M. and Hogness, D., Proc. Nat. Acad. Sci., USA, 72:3961 (1975). Again the $J_L$ and Kappa murine probes were used to identify the recombinant pBR322 plasmid, designated as pMLCE-10 which was deposited under the Budapest Treaty with the American Type Culture Collection on March 1, 1988 (ATCC Deposit #67639). A restriction site and function map of plasmid pMLCE-10 is presented in Figure 1 of the accompanying drawings.

E. Isolation of Human DNA

Whole blood was obtained from individuals homozygous for the human gamma haplotypes fbn and azg in 30 ml samples. Buffy coat cells were harvested from the blood samples by centrifugation at 2500 rpm in a Sorvall GLC-2B at 22°C. The buffy coat cells were removed with a pasteur pipet and washed once with PBS. The cell pellets were resuspended in 500 µl of 10 mM Tris-HCl (pH 8.0) 40 mM NaCl and lysed by the addition of 30 µl 10% SDS and 6 µl 20 mg/ml Protease K (Sigma Chemicals, St. Louis, Missouri). The sample was incubated 15 hours at 37°C. The DNA was extracted twice with an equal volume of phenol:chloroform:isoamyl alcohol (25:24:1), twice more with chloroform:isoamyl alcohol (24:1) and dialyzed against 10 mM Tris-HCl (pH 8.0) 1 mM EDTA. The DNA was then treated for two hours with 50 µg/ml RNAse A (Sigma Chemicals) followed by redigestion with 200 µg/ml Protease K for 1 hour. The DNA was extracted and dialyzed as described above and the concentration determined by $OD_{260}$ to range from 100-200 mg/ml.

F. Construction of a Human Genomic Phage Library

Human DNA of the fbn haplotype (210 µg as isolated in Example 1E) was partially digested with MboI, DNA fragments in the molecular weight range 12-24 kb isolated, and cloned into EMBL-3 phage as described in Example 1B.

G. Isolation of Recombinant Plasmid pHKF-1

In order to isolate the human kappa constant region gene, the EMBL-3 library, as described in Example 1B, was screened with a human kappa probe supplied by Dr. P. Hieter, Johns Hopkins University, Baltimore, Maryland, the sequence of which is available from the NIH Data Base, accession number J00241. A total of $5 \times 10^5$ recombinant phage were screened as described in Example 1C. A single clone was isolated and designated ØHKF-1. Fifteen µl of ØHKF-1 DNA were digested with BamHI and HindIII in React Buffer #3 (Gibco-BRL, Gaithersburg, Maryland). A 5.2 kb fragment was isolated on DEAE 81 paper and ligated into the cloning vector pBR322 as described in Example 1D. Ampicillin resistant recombinant colonies were again identified using the human kappa probe; a single clone was isolated and designated pHKF-1. pHKF-1 was deposited under the Budapest Treaty with the American Type Culture Collection on March 1, 1988 (ATCC Deposit #67637. A restriction site and function map of plasmid pHKF-1 is presented in Figure 1 of the accompanying drawings.

## H. Construction of Plasmid pHKCE-10 Including CEM 231.6.7 $V_L$ Kappa Gene and Human C Kappa Gene

A 3.8 kb HindlI fragment from pMLCE-10 containing the entire CEM 231.6.7 variable kappa region was further subcloned into the HindIII site of plasmid pHKF-1, described in Example 1E, using the methods described in Example 1D to form a chimeric plasmid having the murine CEM 231.6.7 variable light region fused to a human constant kappa region gene. One μg of pMLCE-10 DNA was digested with HindIII 1 unit/μg using React Buffer #2 (Gibco-BRL, Gaithersburg, Maryland). One μg of pHKF1 was digested in a similar manner. The pMLCE-10 digested DNA was electrophoresed as above and the 3.8 kb HindIII fragment was isolated onto DEAE 81 paper and eluted in 5 μl of TE. One μg (2 μl) of HindIII digested pMLCE-10 were ligated to 600 ng of HindIII digested pHKF-1 in the presence of 10x Ligation Buffer, 10 mM ATP and 2 units of T4 DNA ligase of total volume of 10 μl. Ligation was done at 12°C overnight and again HB101 competent cells from BRL were used in the plasmid transformation, described in Example 10. The plasmid designated as pHKCE-10 was identified by restriction mapping of the plasmid DNA. A restriction site and function map of plasmid pHKCE-10 is presented in Figure 2 of the accompanying drawings.

## I. Construction of Plasmid pGCEMK Including Chimeric Light Chain Immunoglobulin Genes

The eukaryotic expression vector containing the murine $V_L$ region fused to the human kappa gene was constructed using the vector pSV2gpt, available from the American type Culture Collection, (ATCC Designation #37145). One μg of pSV2gpt DNA was digested with the restriction enzyme EcoR1 using 1 unit/μg of DNA in Reaction Buffer #3 (Gibco-BRL, Gaithersburg, Maryland). The EcoR1 ends were then made blunt by adding 10 μl of 5 mM each of the 4 deoxyribonucleotides dTTP, dGTP, dCTP and dATP, 2 units of Klenow enzyme and a 10x buffer (.5M Tris HCl pH 7.5; .1 M MgCl$_2$; 10 mM diothiothreitol) in a total 50 μl as described in Molecular Cloning, supra. The reaction went 30 minutes at room temperature and was followed by a ligation reaction in which phosphorylated ClaI linkers (2 μg) (New England BioLabs, Beverly, Massachusetts) were ligated to the 500 ng of EcoR1 blunted ended pSV2gpt in order to create a new ClaI site for our future chimeric vector. The Cla I linkers sequence was d(pCATCCGATG). Ligation reactions were carried out as described under Example 1B. Following ligation, excess linkers were removed by electrophoresis of the DNA and isolation of the linear pSV2gpt-Cla fragment onto DEAE 81 paper as in Example 1D. The isolated DNA was self-ligated using reagents described in Example 1B. HB101 competent cells were transformed as in Example 1D and the ampicillin resistant colonies analyzed by restriction enzyme digestion.

The resulting vector, pSV2gpt-Cla, was then digested with ClaI and BamHI restriction enzymes (1 unit/μg of DNA). The chimeric vector pHKCE-10 was also digested with these two enzymes. The 4.5 kb pSV2gpt Cla-Bam fragment and the 9 kb Cla-Bam pHKCE-10 fragment were isolated on DEAE 81 paper as described in Example 1D. A standard ligation reaction as described above was done using 375 ng of the 9 kb fragment insert DNA and 200 ng of the 4.5 kb vector DNA. Following transformation of HB101, a recombinant plasmid, designated as pGCEMK, was identified by restriction mapping of the plasmid DNA. This plasmid, which is the chimeric expression vector used to produce the CEM chimeric light chain polypeptide, is shown with restriction sites in Figure 2.

## J. Isolation of the Recombinant Phage ØMHCE-30 Including CEM 231.6.7 $V_h$ Gene

In order to identify the CEM 231.6.7 gamma chain, the EMBL-3 library described in Example 1B was screened with two murine heavy chain probes, one representing the $J_h$ 3-4 regions was obtained from Dr. Phil Tucker, University of Texas, Dallas, Texas, and one representing sequences in the murine gamma-1 gene. This latter probe was comprised of the sequences 5'-CTG-TAC-ATA-TGC-AAG-GCT-TAC-AAC-CEC-AAT-3' as determined from the General Bank Data Base (NIH Accession #J00453) and was synthesized by Molecular Biosystems, Inc. (San Diego, CA). A total of $4.8 \times 10^5$ recombinant phage plaques were screened in duplicate using these two probes in order to identify the clone containing both the $J_h$ region and the gamma region. Again, as with the kappa clone, a phage containing sequences for these two regions indicates that that DNA has been rearranged, thus identifying the expressed immunoglobulin gene in the cell line CEM 231.6.7. A single CEM 231.6.7 clone, was selected on the basis of its being rearranged and was designated ØMHCE-30.

### K. Construction of pMHCE-30 Including CEM 231.6.7 $V_h$ Gene

By restriction mapping, a 5.0 kb Sst I fragment from ØMHCE-30 was identified which contained the heavy chain variable region sequences as well as the major intron containing the murine heavy chain enhancer. To subclone this fragment into a plasmid, 10 μg of the page DNA was digested with the restriction enzyme Sst I in Reaction Buffer #2 (BRL-Gibco, Gaithersburg, Maryland) and the 5.6 kb fragment was isolated following electrophoresis by the DEAE 81 method described in Example 1D. The Bluescript vector M13-SK+, commercially available from Stratagene, Inc. (La Jolla, CA) was also digested with Sst I. The vector and isolated 5.6 kb insert DNA was ligated at a ratio of 1:10 in a total of 10 μl as detailed in Example 1B. Following transformation of HB101 competent cells, the correct recombinant was identified by restriction digest mapping, and designated as pMHCE-30. pMHCE-30 was deposited under the Budapest Treaty with the American Type Culture Collection on March 1, 1988 (ATCC Deposit #67640). A restriction site and function map of plasmid pMHCE-30 is presented in Figure 3 of the accompanying drawings.

### L. Isolation of Human DNA

Human DNA was isolated as described and detailed in Example 1E above.

### M. Construction of a Human Plasmid Library

Human DNA of the azg haplotype was digested as 10 μg aliquots using 30 units each of the restriction endonucleases BamHI and HindIII in Reaction Buffer #3 (Gibco-BRL, Gaithersburg, Maryland) (50 mM Tris-HCl pH 8.0, 10 mM $MgCl_2$, 100 mM NaCl) in a total volume of 200 μl. The digested fragments were concentrated to 20 μl by ethanol precipitation and separated on a 0.6% low gelling temperature agarose (FMC) gel run for 15 hours at 50 mAmps. DNA fragments in the size range 6-7 kb were excised from the gel. The cloning vector pUC 18 (described by Yanisch-Perron C., et al., Gene, 33:103 (1985)) was also digested with BamHI and HindIII as described above. The human DNA fragments (150 ng) were ligated into the pUC 18 vector (50 ng) (New England BioLabs, Beverly, Massachusetts) in a total reaction volume of 400 μl containing 30 mM Tris-HCl (pH7.6), 10 mM $MgCl_2$, 5 mM dithiothreitol, 1 mM ATP, and 1 μl T4 DNA ligase (2 units, for 72 hours at 15° C. Half (100 ng) of the ligated DNA sample was used to transform 500 μl of freshly prepared competent E. coli M15 cells and the resulting transformants were plated onto X-gal, IPTG, AMP plates (4 μg/ml X-gal, 2 μg/ml IPTG, 100 μg/ml Ampicillin).

### N. Isolation of Recombinant Plasmid pHG1Z

Ampicillin resistant pUC18 colonies containing recombinant human DNA were screened by the method described in Example 1 using a human Gamma 2 probe supplied by T. Honjo, University of Osaka, Japan and described by Takahashi, N. et al., Cell., 29:671-679 (1982). A clone containing a 7.5 kb insert corresponding to the human Gamma 1 gene was identified and designated HyHG1. This same 7.5 kb HindIII-BamHI fragment containing the human gamma constant region gene was subsequently re-cloned into pBR322 using the same methodology as described in Example 1G. The pBR322 vector containing the human gamma 1 gene was designated pHG1Z and was deposited under the Budapest Treaty with the American type Culture Collection on March 1, 1988 (ATCC Deposit #67638). A restriction site and function map of plasmid pHG1Z is presented in Figure 3 of the accompanying drawings.

### O. Construction of pHGCEM-30 Including CEM 231.6.7 $V_h$ Gene and Human Gamma-1 Gene

The murine variable heavy chain region was fused to the human gamma-1 gene in the following manner. Ten $\mu$g of pMHCE-30 was digested with ClaI (1 unit/$\mu$g) and then partially digested with HindIII to give a 5.3 kb fragment containing $V_h$ and the major intron. Partial digests were performed by using only .1 unit/$\mu$g of DNA and a digestion time of 1 hour at 37°C. One $\mu$g of the plasmid pHGZ-1, described in Example 1N containing the human gamma 1 gene was also digested with ClaI and HindIII. The 5.3 kb fragment from pMHCE-30 was isolated from a TBE gel using the DEAE 81 protocol as described in Example 1D. This fragment was ligated into the Cla-Hind site of pHGZ-1 by using 500 ng of the insert and 200 ng of the vector DNA in a ligation mixture of 10 $\mu$l total volume. Ligation reactions were carried out as detailed in Example 1B. The recombinant plasmids resulting from transformation of HB101 were analyzed by restriction digest mapping in order toidentify a plasmid containing the murine $V_h$ region fused to a human gamma 1 gene, and was designated pHGCEM-30. A restriction site and function map of plasmid pHGCEM-30 is presented in Figure 4 of the accompanying drawings.

P. Construction of pNCEMG1 Including Chimeric Heavy Chain Immunoglobulin Genes

The chimeric Ig gene was inserted into the eukaryotic expression vector essentially as detailed in Example 1H. The vector used was pSV2neo, available from The American Type Culture Collection (ATCC Designation #37149). A ClaI site was added to this vector exactly as described for the pSV2gpt vector. Following this, 1 $\mu$g of pSV2neo-Cla DNA was digested with the enzymes ClaI and BamHI using 1 unit/$\mu$g of DNA. The plasmid pHGCEM-30 was digested with ClaI and then partially digested with BamHI (.1 unit/$\mu$g) in order to obtain a fragment of 12.7 kb which contained the chimeric $V_h$ and gamma 1 regions. This fragment was isolated on DEAE 81 paper and eluted in 10 $\mu$l of TE buffer. The ligation was done using 50 ng of vector DNA, 400 ng of the 12.7 kb insert DNA, 10x ligation buffer, 10 mM ATP and T4 DNA ligase, as in Example 1B at 12°C overnight. HB101 cells were transformed and the correct recombinant plasmid constituting the chimeric expression vector pNCEMG1 was identified. pNCEMG1, which constitutes the recombinant expression vector used to express chimeric heavy chain immunoglobulin genes and is shown with restriction sites, in Figure 4.

Q. Construction of Plasmid pNCEMKG1

Plasmid pGCEMK was digested with restriction enzymes ClaI and BamHI and the approximately 8.9 kb ClaI/BamHI restriction fragment was isolated substantially as hereinbefore described. In an analogous manner, plasmid pNCEMG1 was also digested with restriction enzymes ClaI and BamHI and the approximately 17.6 kb ClaI/BamHI restriction fragment was purified. The about 8.9 kb ClaI/BamHI restriction fragment of plasmid pGCEMK was then ligated into the about 17.6 kb Cla/BamHI restriction fragment of plasmid pNCEMG1 in substantial accordance with the teaching of Example 1B. After transformation into E. coli, re-isolation of plasmid, and restriction mapping, those plasmids which demonstrated the proper restriction maps were designated plasmid pNCEMKG1. A restriction site and function map of plasmid pNCEMKG1 is presented in Figure 5 of the accompanying drawings.

Example 2

DNA Sequencing of Cloned Genes

Sequencing of the cloned CEM variable light and heavy chain genes was accomplished by standard procedures for both double and single stranded templates using the protocols provided by the sequencing kit Sequenase, commercially available from U.S. Biochemicals (Cleveland, Ohio), and the Bluescript/DNA Sequencing System, commercially available from Stratagene, Inc. (La Jolla, CA). From the DNA sequences obtained for the cloned CEM variable light and heavy region genes, the amino acid sequences of the polypeptides encoded for were deduced by a computer software program, MAPSEQ, commercially available from DNAStar, (Madison, Wisconsin).

## Example 3

A. Construction of Plasmid pGCHAG1

Plasmid pUCVHlnc-1A comprises the gene which encodes the murine variable region of heavy metal-binding monoclonal antibody CHA255.5. Plasmid pUCVHlnc-1A can be conventionally isolated from E. coli K12 HB101/pUCVHlnc-1A, a strain made part of the permanent stock culture collection of the NRRL under accession number NRRL B-18433. Plasmid pHG1Z comprises the human gamma gene and is available from the ATCC under the accession number ATCC 67638. About 1 μg of plasmid pUCVHlnc-1A was digested with restriction enzyme HindIII and the approximately 3.4 kb HindIII restriction fragment was isolated. About 1 μg of plasmid pHG1Z was also digested with restriction enzyme HindIII, then treated with Bacterial Alkaline Phosphatase according to procedures wellknown in the art. The about 3.4 kb HindIII fragment of plasmid pUCVHlnc-1A was then ligated into the HindIII-digested, phosphatased plasmid pHG1Z to form plasmid pHG1-CHA. Restriction site and function maps of plasmids pUCVHlnc-1A and pHG1-CHA are presented in Figure 8 of the accompanying drawings.

Plasmid pSV2gpt-Cla (constructed in Example 1) was digested with restriction enzymes ClaI and BamHI and then about 5.0 kb ClaI/BamHI restriction fragment was isolated. In a likewise manner, plasmid pHG1-CHA was also digested with restriction enzymes ClaI and BamHI and the approximately 10.5 kb restriction fragment was isolated. The about 5.0 kb ClaI/BamHI restriction fragment of plasmid pSV2gpt-Cla was ligated to the about 10.5 kb ClaI/BamHI restriction fragment of plasmid pHG1-CHA to form plasmid pGCHAG1. A restriction site and function map of plasmid pGCHAG1 is presented in Figure 9 of the accompanying drawings.

B. Construction of Plasmid pGCHAK

About 1 μg of plasmid pMLCH-1 (from E. coli K12 HB101/pMLCH-1 NRRL B-18432) was digested for three minutes with restriction enzyme BamHI. After an ethanol precipitation the BamHI ends were made blunt by adding 10 μl of 5 mM each of the four deoxyribonucleotides dTTP, dGTP, dATP and dCTP, two units of Klenow enzyme and 5 μl of 10x Buffer (.5 M Tris-HC1 (pH 7.5), .1 M MgCl₂ and 10 mM DTT) in a total of 50 μl reaction volume. After 30 minutes at 37°C, the reaction was stopped by a phenol chloroform reaction and the DNA was self-ligated and transformed into E. coli cells. Those plasmids which demonstrated a deletion of the BamHI site were designated plasmid pMLCH1dB. Restriction site and function maps of plasmids pMLCH1 and pMLCH1dB are presented in Figure 6 of the accompanying drawings.

Plasmid pMLCH2 was next constructed by digesting plasmid pMLCH1dB with restriction enzymes BamHI and HindIII and isolating the about 5.75 kb CHA lambda gene region. This fragment was then ligated into BamHI/HindIII digested plasmid pBR322 to form plasmid pMLCH2. Plasmid pMLCH2 was then digested with restriction enzymes ClaI and BamHI and the approximately 5.75 kb restriction fragment was isolated and ligated into the ClaI/BamHI digested vector fragment of plasmid pSV2gpt-Cla (constructed in Example 1) to form plasmid pGCHA.

Plasmid pGCHA was digested with restriction enzyme BamHI and the approximately 11.2 kb restriction fragment was isolated. Plasmid pHKF1 (available from the ATCC under the accession number 67637) was digested with restriction enzyme HindIII, filled in with Klenow, phosphorylated BamHI (NEB) linkers were added, then the vector was cut with BamHI, and the about 5.2 kb restriction fragment was isolated. The about 11.2 kb BamHI fragment of plasmid pGCHA was then ligated with the about 5.2 kb BamHI restriction fragment of plasmid pHKF1 to form plasmid pGCHAK, which comprises the gene which encodes the murine lambda CHA variable region joined to the gene which encode the human gamma region. A restriction site and function map of plasmid pCHAK is presented in Figure 7 of the accompanying drawings.

C. Construction of Plasmid pGCHAKG1

About 1 μg of plasmid pGCHAK was digested with restriction enzyme BamHI and the BamHI site was ligated to ClaI linkers.

15

```
5'-GATCCGGGATCGATGGGG-3'
   |||||||||||||||
3'-GCCCTAGCTACCCCCTAG-5'
```

in substantial accordance with the teaching of Example 1I). Following ligation, transformation and re-isolation, the resultant plasmid was designated plasmid pGCHAK-Cla. Plasmid pGCHAK-Cla was digested with restriction enzyme ClaI and the approximately 10.85 kb restriction fragment was isolated in accordance with prior teachings. Plasmid pGCHAG1 was digested with restriction enzyme ClaI and the approximately 15.05 kb restriction fragment was isolated. The about 10.85 kb ClaI restriction fragment of plasmid pGCHAK-Cla was then ligated to the about 15.05 kb ClaI restriction fragment of plasmid pGCHAG. After transformation and re-isolation, those plasmids with the proper restriction maps were designated plasmid pGCHAKG1. A restriction site and function map of plasmid pGCHAKG1 is presented in Figure 10 of the accompanying drawings.

## Example 4

### A. Transfection of SP2/0 Cells with the Chimeric Construct pGCHAKG1

The CHA immunoglobulin plasmid used for transfection was pGCHAKG1 as described in the Example 3 above. The pGCHAKG1 plasmid, is first transfected into SP2/0 hybridoma cells by the electroporation techniques referenced above. The SP2/0-Ag14 cells are grown in media containing 5% FCS and maintained in a log phase of growth for the three days preceeding electroporation. Twenty $\mu$g of the plasmid vector pGCHAKG1 is linearized using the restriction enzyme PvuI (1 u/$\mu$g) and the Reaction Buffer #7 (Gibco-BRL, Gaithersburg, Maryland). At the time of transfection the SP2/0 cells are collected by centrifugation in an IEC clinical centrifuge - 800 rpm 10' room temperature. Cells are then washed 3x in Hanks Buffered Saline Solution (Gibco Laboratories, Grand Island, New York) with 6 mM Dextrose and resuspended at a final concentration of 1.5 x $10^7$ cells/ml. 0.3 mls of cells are aliquoted into cuvettes at a density of 0.5 x $10^6$/.3 ml and the linearized DNA is added. The mixture is maintained on ice 10 minutes. Electroporation is done using the .8 mm gap electrode (P/N 472) and the BTX 100 Transfector (BTX, Inc. San Diego, CA.). Conditions are 1 pulse, 5 m seconds each at 250 volts. The electroporated cells are then resuspended in media at a density of 2 x $10^5$/ml (in T75 flasks) for 72 hours. (37° C 5% $CO_2$). Cells are then plated in the appropriate antibiotic at a density of 5 x $10^4$/ml in 24 well plates; SP2/0 cells containing pGCHAKG1 were plated in HMAX 1.0 Media (50 ng/ml Hypoxanthine, 250 ng/ml Mycophenolic Acid and 50 $\mu$g/ml Xanthine), available from Sigma, St. Louis, Missouri, at 1 $\mu$g/ml. Two hundred $\mu$l of supernatant is collected from each well which contained HMAX resistant colonies. This supernatant is then assayed for the presence of a human kappa constant region and a human gamma constant region which would indicate expression of the chimeric immunoglobulin genes of pGCHAKG1.

### B. Identification of SP2/0 Cells Secreting Chimeric CHA 255

Transfected SP2/0 cells expressing the chimeric CHA-human Ig genes were identified by a standard enzyme-linked immunosorbent assay (ELISA), as described by ENgvall, E. and Perlmann, P., Immunochemistry, 8:871-874 (1971), for human Ig.

The purpose of this assay is to identify those cells secreting the chimeric kappa and gamma chain polypeptides coded for by the pGCHAKG1 plasmid vector which was constructed from murine variable regions isolated from the murine hybridoma CHA 255 and fused to the human constant region genes. A 5 $\mu$g/ml solution of goat anti-human gamma chain (Tago #4100) in 10 mM sodium phosphate pH 7-8 is prepared. Each well of a 96 well plate is coated with 50 $\mu$l of this solution. The plates are then incubated overnight at 37° C. Plates are then rinsed thoroughly in $H_2O$ and PBS + 0.1% Tween (w/v). Fifty $\mu$l of the supernatant fractions are added to each well, and incubated for 2 hours at room temperature. Plates are again rinsed as detailed above. A goat anti-human kappa chain alkaline phosphatase conjugate (Tago #2496) is diluted 1:1000 in the same medium as the supernatant material. 100 $\mu$l are added per well and allowed to incubate for 1 hour at room temperature. Plates are rinsed as above. The alkaline phosphatase substrate is prepared as per package instruction, one tablet per 3 ml of distilled $H_2O$ and 150 $\mu$l of this substrate is added to each well and allowed to incubate 30 minutes at 37° C. The reaction is quenched with 50 $\mu$l of 500 mM EDTA and then the absorbance is read at 405 nM. Those supernatants showing the highest levels of Ig expression are identified and the cells from the corresponding wells are pooled and expanded for introduction of the bifunctional chimeric construct pNCEMKG1.

Cells which were expressing high levels of CHA Ig chain are further assayed for production of immunoglobulin which specifically reacts with metal chelates. The assay procedures used for the detection of antibodies directed against metal chelates are standard solid phase radioimmunoassays, essentially as described by Wang, R., et al., Immunol. Methods, 18:157-164 (1977); Reardon et al., Nature 316:265-268 (1985); and Meares and David, U.S. Patent No. 4,722,892, issued February 2, 1988.

The following reagents are added to wells of a microtiter plate and incubated overnight at room temperature with mixing: 25 $\mu$l cell culture supernatent, 50 $\mu$l 111In-EDTA, 20 $\mu$l of Sepharose, bound goat anti-human IgG and 25 $\mu$l cell culture media. Immune complexes bound to the Sepharose-anti-human IgG are collected onto paper filters. Filters are counted in a gamma counter. The radioimmunoassays resulted in the identification of clones which secreted chimeric CHA antibodies. These clones are selected for transfection with plasmid pNCEMKG1.

## C. Transfection of Chimeric CHA Producing Cells with the CEM Chimeric Construct pNCEMKG1

The CEM immunoglobulin plasmid used for transfection into SP2/0 cells was pNCEMKG1, derived from constructs as detailed in Example 1. The pooled populations of cells expressing the chimeric CHA genes are next electroporated with the plasmid constructs containing the chimeric CEM genes. As for the CHA gene electroporation the SP2/0 chimeric CHA producing cells (SP2/0-K) are maintained at log phase of growth for the three days preceeding the electroporation. Twenty micrograms of the plasmid DNA pNCEMKG1 are linearized with the enzyme PvuI in React buffer #6 (Gibco-BRL, Gaithersburg, Maryland). Cells are collected, washed and resuspended at a density of $1.5 \times 10^6$ cells/ml as detailed in Example 2A. The DNA is added and the mixture held on ice for 10 minutes preceeding the electroporation. Conditions used are 1 pulse at 5 m seconds, 250 volts. Cells were plated at $2.5 \times 10^5$/ml in HH2 (or any other commercially available media) 5% FCS plus HMAX 1.0 for 72 hours at 37° C, 5% $CO_2$. Following this, these cells were plated at $5 \times 10^4$/ml in 24 well plates in medium containing HMAX 1.0 and G418 antibiotic (Geneticin, Gibco-BRL, Gaithersburg, Maryland) at an active concentration of 500 $\mu$g/ml. Selection was maintained for 14 days at which time those wells with HMAX/G418 resistant colonies are identified for further analysis.

## Example 5

## Identification and Analysis of SP2/0 Cells Secreting CEM/CHA Bifunctional Chimeric Antibody

### A. Antigen Binding

Screening assays were performed to identify transfected SP2/0 cells which expressed both the chimeric CEM light and heavy chain immunoglobulin genes, producing antibody which binds CEA. The assay procedures used for the detection of antibodies directed against CEA were standard solid phase radioimmunoassays, essentially as described by Wang, R., et al., Immunol. Methods, 18:157-164 (1977).

The following reagents were added to wells of a microtiter plate and incubated overnight at room temperature with mixing: 25 $\mu$l cell culture supernatant, 50 $\mu$l 125I-CEA (affinity purified), 20 $\mu$l of Sepharose bound goat anti-human IgG and 25 $\mu$l cell culture media. Immune complexes bound to the Sepharose-anti-human IgG were collected onto paper filters. Filters were counted in a gamma counter. The radioimmunoassays resulted in the identification clones which were expressing anti-bodies which specifically reacted with CEA.

### B. Antibody Affinity

Affinity assays were performed to determine the Ka of bifunctional chimeric antibody CEM/CHA for CEA. The affinity of bifunctional chimeric antibody CEM/CHA for CEA was determined by standard radioimmunoassay procedures, as described in Example 4A, and Scatchard analysis as described by Scatchard, G., Ann. New York Acad. Sci., 51:660 (1949).

Antigen binding assays were done as described in Example 5A. To generate the inhibition curve, 25µl volumes of CEA were added to each reaction, substituting for the 25 µl of cell culture media. The mass of competitor added ranged from 1-100 ng. For the Scatchard analysis, a plot of bound/free vs bound antigen permitted calculation of the affinity constant as defined by the megative of the slope of the line. Affinities of the chimeric antibodies were at least comparable to those of the murine antibody counterparts from which the chimeric antibodies of the invention were derived.

In an analogous manner, the ka of bifunctional chimeric antibody CEM/CHA for In-EDTA chelates was also determined.

Antibody assays were performed to determine the Ka of the instant chimeric antibody for the indium (III) complex of EOTUBE. EOTUBE is a derivative of p-(aminobenzyl) EDTA and is described in L.D. Anderson, J.M. Frincke, and D.L. Meyer, U.S. Patent No. 151855, filed February 3, 1988, (European Patent Application No. 89301012.4) herein incorporated by reference.

The synthesis of EOTUBE and its use in a standard Scatchard are set forth below.

## 1. Synthesis of EOTUBE

Specifically, EOTUBE is EDTA substituted at one of the internal ethylene carbons (S stereochemistry at that substituted carbon) through the benzylic carbon of an N-(2-hydroxyethyl)-N$'$-(p-benzyl)thiourea moiety. The synthesis of EOTUBE was performed excluding metal ions as much as possible. (For example, all glassware was washed with 6M HCl, and only dionized water was used).

(S)-p-nitrobenzyl EDTA was prepared and converted to (S)-4-isothiocyanatobenzyl EDTA (hereinafter abbreviated as ITCBE) as described on U.S. Patent No. 4,622,420, and Meares, C.F., Anal. Biochem 142: 68-75 (1984). The lyophilized ITCBE was resuspended in 0.3M HCl (Ultrex, J.T. Laker, Phillipsburg, N.J.) to a final concentration of roughly 50 mM. This solution was stored at -70$^\circ$ C. Unless indicated otherwise, all reactions were done in aqueous solution as 40$^\circ$ C.

2.5 ml of 20 mM ITCBE was added to 1.35 ml of 200 mM ethanolamine and the pH adjusted to 11.0 with 10 N NaOh. The volume was adjusted to 5 ml with water and the mixture allowed to react for 15 minutes, at which time it was checked by HPLC analysis. All of the ITCBE was converted to EOTUBE, with a retention time of 3.6 minutes on a HPLC (G18 column, eluted with a linear gradient of aqueous buffer of 50 mM triethylammonium acetate to neat methanol on a Hewlett-Packard 1090 instrument).

The product was purified by anion exchange chromatography on an 11 ml column of DEAD Sephadex A-25, eluted with a 110 ml gradient of 0.1 to 1 M ammonium formate, pH 6. (The column was monitored at 280 nm.) Fractions containing the product were pooled and lyophilized. The product has an absorbance maximum at 246 nm with an extinction coefficient of 18000.

The structure was purified by carbon 13 NMR spectroscopy (on a Varian Instruments Model XL-300 300 Mhz instrument. The spectrum was run deuterated water.) The peak corresponding to the carbon in the isothiocyanate moiety in ITGBE was at 139 ppm, and was replaced by a peak at 182 ppm in EOTUBE. This peak corresponds to the carbon in the thiourea linkage. The aromatic region (128-138 ppm) of the spectrum of ITCBE shows four peaks, while that of EOTUBE shows three. In the aliphatic regions, there are five peaks in common for ITCBE and EOTUBE, and an additional two peaks at 64 and 49 ppm in the EOTUBE spectrum. The latter peaks correspond to the carbons adjacent to the hydroxyl and thiourea moieties, respectively.

## AFFINITY PROCEDURE

### I. Radiolabel of Chelate

A. Label 9.9540 x 10$^{-7}$ mmoles of EOTUBE with 600 µCi $^{111}$In.
    1. Without introducing any other metal add the following to a metal-free tube:
a. 63 µl 0.0158 mM metal-free EOTUBE

b. 63 $\mu$l 0.26 M metal-free ammonium citrate pM = 6.0

c. 600 $\mu$Ci $^{111}$In (1.30 x 10$^{-9}$ mmoles)

    2. Incubate overnight at room temperature.

    B. Load step A. with enough cold indium to give a 1.05 molar ratio of indium (both $^{111}$In and $^{ground}$ $^{state}$In) over EOTUBE

        1. To the tube from above (I.A.@.) add 10.2 $\mu$l 0.012 mM $^{ground\ state}$InCl$_3$ (1.044 x 10$^{-6}$ mmoles).

        2. Incubate 4 hours at room temperature.

    C. Perform Thin-Layer Chromatography analysis to test incorporation of Indium.

        1. Spot 0.5 $\mu$l of the labeled sample IN duplicate lanes 1 cm from the bottom of a silica gel plate 10.5 inches long marked off in 0.25 inch strips to make lanes.

        2. Allow the sample spots to dry

        3. Place plate in a TLC tank with a 10% ammonium acetate:methanol (1:1) solvent system.

        4. Develop plate to the 9 cm mark.

        5. Remove plate and allow to dry.

        6. Cut each lane of the plate into three sections.

a. The bottom to the 2 cm mark is the origin section.

b. 2 cm-3.5 cm is the middle section.

c. 3.5 cm-top is the tail section.

        7. Count the cpms of each section for both lanes.

        8. The percent at the tail for each lane is equal to the present indium incorporated into the chelate.

    D. If indium incorporated greater than 90% dilute EOTUBE to 4.40 pg/$\mu$l in PBS pH-7.5 for use in assay.

II. Cold Competitor Preparation

    A. Cold indium load 5.530 x 10$^{-4}$ mmoles EOTUBE with a molar ratio of 1.02 x InCl$_3$ over EOTUBE.

    1. Without introducing any contaminating metal add the following to a metal-free tube:

a. 70 $\mu$l 7.9 mM metal-free EOTUBE (5.530 x 10$^{-4}$ mmoles).

b. 70 $\mu$l 0.26 M metal-free ammonium citrate pH = 6.0

c. 55.3 $\mu$l 10.2 mM $^{ground\ state}$InCl$_3$ (5.641 x 10$^{-4}$ mmoles).

    2. Incubate 4 hours at room temperature.

    B. Dilute to get 1 ml of 0.40 mg/$\mu$l and 1 ml of 0.36 ng/$\mu$l in PBS pH = 7.5 for use in part IV.

III. Antibody Sensitivity Curve

    A. To a 96 well microtiter plate add the following in triplicate:

        1. 25 $\mu$l EOTUBE $^{111}$In$^{ground\ state}$In at 4.4 pg/$\mu$l.

        2. 25 $\mu$l antibody dilution either 20 $\mu$g/ml, 10, 5, 2.5, 1.25, 0.623, 0.313, 0.156, 0.080, 0.020, or 0 $\mu$g/ml.

        3. 50 $\mu$l RPMI with 10% horse serum.

        4. 20 $\mu$l sheep anti-mouse IgG coupled to sepharose beads (March, S.C., Parikh, I., and Guatrecacas, P., Analyt. Biochem 60: 149, (1974) diluted to a concentration so that 20 $\mu$l will bind 0.5 $\mu$g Ab.

    B. Incubate overnight on a rotator at room temperature.

    C. Aspirate contents of wells onto glass-fibre filter paper.

    D. Cut out and count filter discs.

    E. Graph antibody dilution # vs. fraction bound.

    F. Determine what point (dilution #) equals 90% of maximum bound.

    G. Use the determined dilution from above for the scatchard assay.

IV. Scatchard Assay

    A. To a 96 well microtiter plate add the following in triplicate:

        1. 25 $\mu$l EOTUBE $^{111}$In-$^{ground\ state}$In at 4.40 pg/$\mu$l.

        2. 25 $\mu$l EOTUBE-$^{ground\ state}$In at scrial dilutions of the 0.40 ng/$\mu$l and 0.36 ng/$\mu$l made in part IIB. Eleven dilutions each plus a zero giving 24 points with a range of 10 ng/well down to 4.40 pg/well plus a zero. Dilute with RPMI containing 10% horse serum.

        3. 25 $\mu$l RPMI containing 10% horse serum.

4. 25 μl antibody of interest. (concentration determined by sensitivity assay).

5. 20 μl sheep anti-mouse IgG coupled to sepharose beads (same as used in sensitivity curve assay).

B. Incubate overnight on a rotator at room temperature.

C. Aspirate contents of wells onto glass-fiber filter paper.

D. Cut out and count filter paper discs.

E. Plot Molar Bound vs. Bound/Free.

F. Take linear regression of part of the curve that is a straight line. The negative of the slope is the Ka.

## C. Assay for Bifunctional Activity of Bifunctional Chimeric Antibody CEM/CHA

Polystyrene beads were coated with monoclonal antibody CEV124, which specifically binds CEA. CEA was then added to the coated beads and allowed to incubate for one hour at 37° C, then the beads were rinsed with PBS. Supernatent containing bifunctional chimeric CEM/CHA was then added to the beads, and the reaction was allowed to proceed for one hour at 37° C. After a rinse with PBS, the beads were mixed with 111IN-EDTA for one hour at 37° C. After another rinse, immune complexes comprising antibody CEM/CHA were collected onto paper filters which were counted in a gamma counter. Transfected SP2/0 cells which produced bifunctional chimeric CEM/CHA, wherein one arm binds to CEA and the other arm binds to metal chelates, were identified and subcloned. Following adaptation of the cells to adapt them to serum-free media (HH2), levels of total Ig expression as high as 60 μg/ml/$10^6$ cells were attained.

## Example 6

## Construction of an SV40 Enhancerless Cloning System

## A. Construction of Plasmid pSV2gpt(E-)

About 20 μl (10 μg) of plasmid pSV2gpt-Cla (constructed in Example 1I) were mixed with 21 μl water, 5 μl Gibco Reaction Buffer #6, 2 μl restriction enzyme PvuII and 2 μl restriction enzyme SphII, then incubated for 2 hours at 37° C. The reaction mixture was electrophoresed through a 0.5% TBE gel and the PvuII/SphII-digested vector fragment was purified from DEAE 81 paper in substantial accordance with the teaching of Example 1D. To fill in the 3' protruding ends of these restriction sites, about 20 μl of the PvuII/SphII-digested pSV2gpt-Cla vector were mixed with 3 μl 10x T4 polymerase buffer (700 mM Tris, pH 7.4; 100 mM MgCl₂; 50 mM DTT), 3 μl of dNTP mix (0.5 mM each of dATP, dTTP, dCTP and dGTP, pH 7.0), 3 μl of water and 1 μl (5U) of T4 DNA polymerase (BRL). The mixture was incubated at 37° C for 15 minutes, then heated to 70° C for 10 minutes. After a phenol extraction and ethanol precipitation, the DNA was resuspended in 5 μl of TE buffer. About 1 μl (about 500 ng) of this DNA fragment was mixed with 10 μl of water, 5 μl of 5x ligation buffer, 2 μl of 100 μMATP and 2 μl of T4 ligase. After an incubation of 2 hours at room temperature, the ligation mixture was transformed into E. coli HB101 cells in substantial accordance with the teaching of Example 1P. Plasmid DNA was isolated from the trans formants and those plasmids which displayed the proper ~0.3 kb deletion of the SV40 enhancer region were designated plasmid pSV2gpt(E-).

## B. Construction of Plasmid pSV2neo(E-)

An SV40 enhancerless pSV3neo was also constructed. About 20 μl (10 μg) of plasmid pSV2neo-Cla (constructed in Example 1P) was digested with restriction enzymes BamHI and HindIII in a reaction mixture using Gibco Raction Buffer #3, substantially as described above. The approximately 2.3 kb HindIII/BamHI neomycin resistance-conferring gene containing fragment was isolated and purified from DEAE 81 paper following electroelution. In a similar manner, plasmid pSV2gpt(E-) was also digested with restriction enzymes BamHI and HindIII, and the large vector fragment was purified after electrophoresis. The about 2.3 kb HindIII/BamHI neo containing restriction fragment of plasmid pSV2neo-Cla was then ligated into the HindIII/BamHI-digested vector fragment of plasmid pSV2gpt(E-) in substantial accordance with the teaching of the previous paragraph. After transformation into E. coli HB101 and isolation of plasmid DNA, those plasmids which the pSV2gpt(E-) vector backbone ligated with the about 2.3 kb BamHI/HindIII neo fragment of plasmid pSV2neo-Cla are designated plasmid pSV2neo(E-).

## C. Construction of Plasmids pGCEMK(E-) and pGCEMG(E-)

About 10 μg (100 μl) of plasmid pSV2gpt(E-)DNA were mixed with 4 μl of water, 12 μl of Gibco Reaction Buffer #1, 2 μl of restriction enzyme ClaI and 2 μl restriction enzyme BamHI, then incubated overnight at 37° C. After electrophoresis onto DEAE 81 paper, the large vector fragment was purified and resuspended in 10 μl TE. About 20 μl (5 μg) of plasmid pHKCE-10 (constructed in Example 1H) were mixed with 23 μl water, 5 μl of Gibco Reaction Buffer #1 and 2 μl restriction enzyme ClaI. After 5 hours at 37° C, the reaction was extracted with phenol/chloroform and ethanol precipitated. This digested DNA was then resuspended in 25 μl of water and mixed with 3 μl of Gibco Reaction Buffer #3 and 2 μl Restriction enzyme BamHI. Following a 2 hour incubation at 37° C, the digested DNA was electrophoresed and the approximately 9.0 kb murine variable, human construct kappa-encoding ClaI/BamHI restriction fragment was purified from DEAE 81 paper. This approximately 9.0 kb fragment was then ligated into the ClaI/BamHI-digested plasmid pSV2gpt(E-) and transformed into E. coli cells as previously described. After plasmid isolation, those plasmids which comprise the correct restriction maps are designated plasmid pGCEMK(E-).

In a similar fashion, about 40 μl (5 μg) of plasmid pHGCE-30 (constructed in Example 10) were mixed with 3 μl of water, 5 μl of Gibco Reaction Buffer #1 and 2 μl restriction enzyme ClaI. After 5 hours at 37° C, the reaction was extracted with phenol/chloroform and ethanol precipitated. This digested DNA was resuspended in 26 μl of water, 3 μl of Gibco Reaction Buffer #3 and 1 μl of restriction enzyme BamHI. After 2 minutes at 37° C, 15 μl of the reaction mixture was removed and mixed with 1 μl of 250 μm EDTA. After 5 minutes at 37° C, the remaining 15 μl of the reaction mixture was also removed and mixed with 1 μl of 250 μM EDTA. This partial BamHI digestion yielded all possible ClaI/BamHI restriction fragments. After electrophoresis on a 0.5% TBE gel, the approximately 12.7 kb ClaI/BamHI restriction fragment was purified from DEAE 81 paper. This restriction fragment comprises the murine variable, human constant gamma-encoding gene. The approximately 12.7 kb ClaI/BamHI (partial) restriction fragment was then ligated into the ClaI/BamHI-digested plasmid pSV2gpt(E-), then transformed into E. coli. After re-isolating and restriction site mapping, the plasmids which comprise the proper restriction maps are designated plasmid pGCEMG(E-).

## D. Construction of Plasmids pNCEMK(E-) and pNCEMG1(E-)

About 10 μg (100 μl) of plasmid pSV2neo(E-) DNA were digested with restriction enzymes ClaI and BamHI, then the vector fragments were isolated and purified in substantial accordance with the teaching of Example 5C. The approximately 9.0 kb ClaI/BamHI restriction fragment of plasmid pHKCE-10 (isolated in Example 5C), which comprises the murine variable, human constant kappa-encoding genes, was then ligated into the ClaI/BamHI digested vector fragment of plasmid pSV2neo(E-) to form plasmid pNCEMK(E-), all in sub stantial accordance with the teaching of Example 5C. Also in accordance with the teaching of Example 5C, plasmid pNCEMG1(E-) was constructed by ligating the approximately 12.7 kb ClaI/BamHI (partial) restriction fragment of plasmid pHGCE-30 into the ClaI/BamHI digested vector fragment of plasmid pSV2neo(E-). Plasmid pNCEMG(E-) therefore comprises the murine variable, human constant gamma encoding genes on an SV40 enhancerless expression vector.

## E. Construction of Plasmids pGCHAK(E-) and pGCHAG1(E-)

Plasmid pGCHAK(E-) is constructed in substantial accordance with the teaching for the construction of plasmid pGCHAK (Example 3B), except plasmid pSV2gpt(E-) is used rather than plasmid pSV2gpt. Plasmid pGCHAG1(E-) is constructed in substantial accordance with the teaching for the construction of plasmid pGCHAG1 (Example 3A), except plasmid pSV2gpt(E-) is used rather than plasmid pSV2gpt.

## F. Construction of Plasmid pNCEMKG1(E-)

Plasmid pNCEMKG1(E-) is constructed in substantial accordance with the teaching for the construction of plasmid pNCEMKG1 (Example 1Q), except plasmid pNCEMG1(E-) is used rather than plasmid pNCEMG1.

G. Construction of Plasmid pGCHAKG1(E-)

Plasmid pGCHAKG1(E-) is constructed in substantial accordance with the teaching for the construction of plasmid pGCHAKG1 (Example 3C), except plasmid pGCHAG1(E-) is used rather than plasmid pGCHAG1.

H. Transfection of SP2/0 Cells with Plasmids pGCHAKG1(E-) and pNCEMKG1(E-)

Plasmid pGCHAKG1(E-) is first transfected into SP2/0 cells in substantial accordance with the teaching of Example 4. After transfection SP2/0 cells which express the active CHA chimeric are identified by the techniques of Example 5B, these clones are transfected with plasmid pNCEMKG1(E-) to produce cells which will demonstrate high levels of expression of bifunctional chimeric antibody CEM/CHA.

Example 7

Construction of a High Level Expression System for Expression of Heterologous Immunoglobulin Chains

A. Construction of Plasmid p19HANCH

Intermediate plasmid p19HANCH was first constructed by preparing an oligonucleotide polylinker with the DNA sequence comprising:

```
5'-AATTCAGATCTGGTGACCCGCGGTCGACGGGCTGCAGAATATTGCGGCCGCCATGGATCCA-3'
   |||||||||||||||||||||||||||||||||||||||||||||||||||||||||||||||
   3'-GTCTAGACCACTGGGCGCCAGCTGCCCGTCGTCTTATAACGCCGGCGGTACCTAGGTTCGA-5'
```

The linker depicted above was synthesized from single-stranded deoxyoligonucleotides by procedures well known in the art. The single-stranded deoxyoligonucloeitdes can be synthesized with commercially available instruments, such as the Biosearch 8700 DNA Synthesizer marketed by Biosearch, Inc. (San Raphael, CA), which utilizes phosphoramidite Chemistry. Other procedures for synthesizing DNA are also known in the art. The conventional modified phosphotriester method of synthesizing single stranded DNA is described in Itakura et al., 1977, Science 198:1056 and in Crea et al., 1978, Proc. Nat. Acad. Sci. USA 75:5765. In addition, an especially preferred method of synthesizing DNA is disclosed in Hsiung et al., 1983, Nucleic Acid Research 11:3227 and Narang et al., 1980, Methods of Enzymology 68:90.

About 2.5 μg of each of the two oligonucleotide strands were mixed with 50 μl of 2x Annealing Buffer (0.2 M NaCl, 20 mM Tris-HCl (pH 7.8) and 2 mM (EDTA) and 50 μl of water. The reaction mixture was heated to 70°C for 5 minutes then allowed to slowly cool to room temperature so that the two single strands would anneal into one linker segment. Approximately 1 μg of plasmid pUC19 (New England Biolabs) was digested with restriction enzymes EcoRI and HindIII in substantial accordance with earlier teachings. After purification of the about 2.6 kb vector fragment, the synthesized polylinker was ligated into the EcoRI/HindIII digested plasmid pUC19. After transformation into E. coli and re-isolation of plasmid DNA, those plasmids which demonstrated the proper HindIII, SspI, PstI, SstII and EcoRI sites within the linker region were designated plasmid p19HAN.

Plasmid S(-)CHAVL was constructed by first digesting the Bluescript vector, M13(-)SK (Stratagene) with restriction enzymes BamHI and SstI, then isolating the large vector fragment in substantial accordance with earlier teaching. Plasmid pMLCH-1 was next digested with restriction enzymes BamHI and SstI, and the approximately 1100 bp fragment which comprises the gene encoding the murine CHA variable region was isolated. Plasmid pMLCH-1 can be conventionally isolated from E. coli K12 HB101/pMLCH-1, a strain deposited and made part of the permanent stock culture collection of the Northern Regional Research Laboratory, 1815 North University Street, Peoria, IL 61604. E. coli K12 HB101/pMLCH-1 is available under the accession number NRRL B-18432. The approximately 1100 bp BamHI/SstI restriction fragment of plasmid pMLCH-1 was then ligated into the BamHI/SstI digested vector M13(-)SK and the plasmid was transformed into E. coli in substantial accordance with prior examples. Following re-isolation and restriction mapping, those plasmids which contained the proper approximately 1100 bp BamHI/SstI fragment were designated plasmid S(-)CHAVL.

About 1 µg of plasmid S(-)CHAVL was next digested with restriction enzyme PstI in substantial accordance with prior teachings. An approximately 900 bp PstI restriction fragment which comprises the gene encoding the murine CHA variable region was isolated and ligated into plasmid p19HAN, which had first been cut in the polylinker region by restriction enzyme PstI. Following transformation, re-isolation and restriction mapping, those plasmids which comprise the J region of the CHA gene closest to the HindIII site of the polylinker are designated plasmid p19HANCH.

## B. Construction of Expression Vectors pGCHAK-2 and pGCHAK-3

About 5 µg of plasmid pGCEMK (constructed in Example 1) was digested with restriction enzymes ClaI and SspI in substantial accordance with earlier teachings, then the approximately 2.2 kb ClaI/SspI restriction fragment, which comprise the CEM kappa promoter, was purified. In a separate reaction, plasmid pGCEMK was digested with restriction enzymes ClaI and SstII, and the approximately 9.4 kb vector fragment was isolated. Furthermore, plasmid p19HANCH was digested with restriction enzymes SspI and SstII and the approximately 931 base pair restriction fragment which comprises the gene encoding the murine kappa CHA variable region was isolated. In a three part ligation reaction, the approximately 9.4 kb ClaI/SstII vector fragment of plasmid pGCEMK, the approximately 2.2 kb ClaI/SspI CEM-promoter containing restriction fragment of plasmid pGCEMK and the approximately 931 base pair SspI/SstII restriction fragment of plasmid p19HANCH which contains the gene encoding the murine kappa CHA variable region were all ligated together and transformed into E. coli in substantial accordance with prior examples. After plasmid isolation and restriction mapping, those plasmids which display the proper restriction fragment sizes are designated plasmid pGCHAK-2.

In an analogous manner, plasmid pGCEMK(E-) (constructed in Example 6C) was digested with restriction enzymes ClaI and SstII and the approximately 9.2 kb restriction fragment (SV40 enhancerless) was isolated. This fragment was then ligated to the approximately 2.2 kb ClaI/SspI CEM kappa promoter-containing restriction fragment of plasmid pGCEMK and the approximately 931 base pair SspI/SstII restriction fragment of plasmid p19HANCH to form plasmid pGCHAK-3. Plasmid pGCHAK-3 differs from plasmid pGCHAK-2 only in the fact that plasmid pGCHAK-3 lacks the SV40 enhancer.

## C. Construction of Plasmids pGCHAKG1-2 and pGCHAKG1-3

Plasmid pGCHAKG1-2 is constructed in substantial accordance with the teaching for the construction of plasmid pGCHAKG1 (Example 3C), except plasmid pGCHAK-2 is used, rather than plasmid pGCHAK. In an analogous manner, plasmid pGCHAKG1-3 is constructed in substantial accordance with the teaching for the construction of plasmid pGCHAK (Example 3C), except plasmids pGCHAK-2 is used, rather than plasmid pGCHAK and plasmid pGCHAG1(E-) is used rather than pGCHAG1. Plasmid pGCHAKG1-2 comprises the genes encoding the CHA chimeric light chain driven by the CEM kappa promoter as well as the chimeric heavy chain CHA-specific gene. Plasmid pGCHAKG1-3 comprises the CHA chimeric light chain driven by the CEM kappa promoter, as well as the chimeric heavy chain CHA-specific gene, on an SV40 enhancerless vector. Transfection of plasmids pGCHAKG1-2 and pNCEMKG1 into SP2/0 cells will demonstrate increased levels of expression of bifunctional chimeric CEM/CHA, while transfection of SP2/0 with plasmids pGCHAKG1-3 and pNCEMKG1 will demonstrate even higher levels of expression of bifunctional chimeric CEM/CHA.

**Claims**

1. A recombinant DNA compound comprising a first DNA sequence which encodes for the human

23

carcinoembryonic antigen-specific light chain variable region of a chimeric monoclonal antibody and a second DNA sequence which encodes for the human carcinoembryonic antigen-specific heavy chain variable region of a chimeric monoclonal antibody, wherein the antibody light chain variable region amino acid sequence comprises:

Asp - Ile - Val - Met - Thr - Gln - Ser - Gln - Lys Phe - Met - Ser - Thr - Ser - Val - Gly - Asp - Arg Val - Ser - Ile - Thr - Cys - Lys - Ala - Ser - Gln Asn - Val - Arg - Thr - Ala - Val - Ala - Trp - Tyr Gln - Gln - Lys - Pro - Gly - Gln - Ser - Pro - Lys Ala - Leu - Ile - Tyr - Leu - Ala - Ser - Asn - Arg Tyr - Thr - Gly - Val - Pro - Asp - Arg - Phe - Thr Gly - Ser - Gly - Ser - Gly - Thr - Asp - Phe - Thr Leu - Thr - Ile - Thr - Asn - Val - Gln - Ser - Glu Asp - Leu - Ala - Asp - Tyr - Phe - Cys - Leu - Gln His - Trp - Asn - Tyr - Pro - Leu - Thr - Phe - Gly Ala - Gly - Thr - Lys - Leu - Glu - Leu - Lys - Arg

and wherein the antibody heavy chain variable region amino acid sequence comprises:

Asp - Val - Gln - Leu - Val - Glu - Ser - Gly - Gly Gly - Leu - Val - Gln - Pro - Gly - Gly - Ser - Arg Lys - Leu - Ser - Cys - Ala - Ala - Ser - Gly - Phe Thr - Phe - Ser - Asn - Phe - Gly - Met - His - Trp Ile - Arg - Gln - Ala - Pro - Glu - Lys - Gly - Leu Glu - Trp - Val - Ala - Tyr - Ile - Ser - Gly - Gly Ser - Ser - Thr - Ile - Tyr - Tyr - Ala - Asp - Thr Val - Lys - Gly - Arg - Phe - Thr - Ile - Ser - Arg Asp - Asn - Pro - Arg - Asn - Thr - Leu - Phe - Leu Gln - Met - Thr - Ser - Leu - Arg - Ser - Glu - Asp Thr - Ala - Met - Phe - Tyr - Cys - Ala - Arg - Asp Tyr - Tyr - Ala - Asn - Asn - Tyr - Trp - Tyr - Phe Asp - Val - Trp - Gly - Ala - Gly - Thr - Thr - Val Thr - Val - Ser - Ser - Ala.

2. The recombinant DNA compound according to Claim 1 wherein the first DNA strand coding sequence comprises:

GAC - ATT - GTG - ATG - ACC - CAG - TCT - CAA - AAA TTC - ATG - TCC - ACA - TCA - GTA - GGA - GAC - AGG GTC - AGC - ATC - ACC - TGC - AAG - GCC - AGT - CAG AAT - GTT - CGT - ACT - GCT - GTT - GCC - TGG - TAT CAA - CAG - AAA - CCA - GGG - CAG - TCT - CCT - AAA GCA - CTG - ATT - TAC - TTG - GCA - TCC - AAC - CGG TAC - ACT - GGA - GTC - CCT - GAT - CGC - TTC - ACA GGC - AGT - GGA - TCT - GGG - ACA - GAT - TTC - ACT CTC - ACC - ATT - ACC - AAT - GTG - CAA - TCT - GAA GAC - CTG - GCA - GAT - TAT - TTC - TGT - CTG - CAA CAT - TGG - AAT - TAT - CCG - CTC - ACG - TTC - GGT GCT - GGG - ACC - AAG - CTG - GAG - CTG - AAA - CGG

and wherein the second DNA strand coding sequence comprises:

GAT - GTG - CAG - CTG - GTG - GAG - TCT - GGG - GGA GGC - TTA - GTG - CAG - CCT - GGA - GGG - TCC - CGG AAA - CTC - TCC - TGT - GCA - GCC - TCT - GGA - TTC ACT - TTC - AGT - AAC - TTT - GGA - ATG - CAC - TGG ATT - CGT - CAG - GCT - CCA - GAG - AAG - GGA - CTG GAG - TGG - GTC - GCA - TAC - ATT - AGT - GGT - GGC AGT - AGT - ACC - ATC - TAC - TAT - GCA - GAC - ACA GTG - AAG - GGC - CGA - TTC - ACC - ATC - TCC - AGA GAC - AAT - CCC - AGG - AAC - ACC - CTC - TTC - CTG CAA - ATC - ACC - AGT - CTA - AGG - TCT - GAG - GAC ACG - GCC - ATG - TTT - TAC - TGT - GCA - AGA - GAT TAC - TAC - GCT - AAC - AAC - TAC - TGG - TAC - TTC GAT - GTC - TGG - GGC - GCA - GGG - ACC - ACG - GTC ACC - GTC - TCC - TCA - GCC.

3. The recombinant DNA compound according to Claim 1 or 2 wherein the DNA construct further comprises a third DNA strand sequence which codes for the light chain constant region of the chimeric monoclonal antibody and a fourth DNA strand sequence which codes for the heavy chain constant region of the chimeric monoclonal antibody.

4. The recombinant DNA compound according to any of Claims 1, 2 or 3 wherein the first and second DNA strand coding sequences are derived from a murine hybridoma.

5. The recombinant DNA compound according to Claim 4 wherein the murine hybridoma is CEM 231.6.7.

6. The recombinant DNA compound according to Claim 3 wherein the third and fourth DNA strand coding sequences are derived from the human lymphocyte.

7. The recombinant DNA compound of Claim 6 that is plasmid pNCEMKG1 as shown in Figure 5.

8. The recombinant DNA compound of Claim 6 that is plasmid pNCEMKG1(E-) as described in Example 6F.

9. A recombinant DNA compound comprising a first DNA sequence which encodes for the chelate-specific light chain variable region of a chimeric monoclonal antibody and a second DNA sequence which encodes for the chelate-specific heavy chain variable region of a chimeric monoclonal antibody, wherein the antibody light chain variable region amino acid sequences comprises:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

and wherein the antibody heavy chain variable region amino acid sequence comprises:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala.

10. The recombinant DNA compound according to Claim 8 wherein the first DNA strand coding sequence comprises:

CAG GCT GTT GTG ACT CAG GAA TCT GCA CTC ACC ACA TCA CCT GGT GAA ACA GTC ACA CTC ACT TGT CGC TCA AGT ACT GGG GCT GTT ACA ACT AGT AAC TAT GCC AAC TGG GTC CAA GAA AAA CCA GAT CAT TTA TTC ACT GGT CTA ATA GGT GGT ACC AAT AAC CGG GCT CCA GGT GTT CCT GCC AGA TTC TCA GGC TCC CTG ATT GGA GAC AAG GCT GCC CTC ACC ATC ACA GGG GCA CAG ACT GAA GAT GAG GCA AGA TAT TTC TGT GCT CTA TGG TAC ACC AGC CTC TGG GTA TTC GGT GGA GGA ACC AAA CTG ACT GTC CTA GGG

and wherein the second DNA strand coding sequence comprises:

GAA GTG ACG CTG GTG GAG TCT GGG GGA GAC TCA GTG AAG CCT GGA GGG TCC CTG AAA CTC TCC TGT GCA GCC TCT GGA TTC ACT TTA AGT GGT GAA ACC ATG TCT TGG GTT CGC CAG ACT CCG GAG AAG AGG CTG GAG TGG GTC GCA ACC ACT CTT AGT GGT GGT GGT TTC ACC TTC TAT TCA GCC AGT GTG AAG GGT CGT TTC ACC ATC TCC AGA GAC AAT GCC CAG AAC AAC CTC TAT CTA CAA CTG AAT AGT CTG AGG TCT GAG GAC ACG GCC TTG TAT TTC TGT GCA AGT CAT CGG TTT GTT CAC TGG GGC CAC GGG ACT CTG GTC ACT GTC TCT GCA GCC.

11. The recombinant DNA compound according to Claim 1 or 2 wherein the DNA construct further comprise a third DNA strand sequence which codes for the light chain constant region of the chimeric monoclonal antibody and a fourth DNA strand sequence which codes for the heavy chain constant region of the chimeric monoclonal antibody.

12. The recombinant DNA compound according to any of Claims 9, 10 or 11 wherein the first and second DNA strand coding sequences are derived from a murine hybridoma.

13. The recombinant DNA compound according to Claim 12 wherein the murine hybridoma is CHA 255.5.

14. The recombinant DNA compound according to Claim 11 wherein the third and fourth DNA strand coding sequences are derived from a human lymphocyte.

15. The recombinant DNA compound of Claim 14 that is plasmid pGCHAKG1 as shown in Figure 10.

16. The recombinant DNA compound of Claim 14 that is plasmid pGCHAKG1(E-) as described in Example 6G.

17. The recombinant DNA compound of Claim 14 that is plasmid pGCHAKG1-2 as described in Example 7B.

18. The recombinant DNA compound of Claim 14 that is plasmid pGCHAKG1-3 as described in Example 7B.

19. A transformed host cell which secretes bifunctional chimeric CEM/CHA.

20. The host cell of Claim 19 that is SP2/0/pNCEMKG1/pGCHAKG1.

21. The host cell of Claim 19 that is SP2/0/pNCEMKG1/pGCHAKG1(E-).

22. The host cell of Claim 19 that is SP2/0/pNCEMKG1/pGCHAKG1-2.

23. The host cell of Claim 19 that is SP2/0/pNCEMKG1/pGCHAKG1-3.

24. The host cell of Claim 19 that is SP2/0/pNCEMKG1(E-)/pGCHAKG1.

25. The host cell of Claim 19 that is SP2/0/pNCEMKG1(E-)/pGCHAKG1(E-).

26. The host cell of Claim 19 that is SP2/0/pNCEMKG1(E-)/pGCHAKG1-2.

27. The host cell of Claim 19 that is SP2/0/pNCEMKG1(E-)/pGCHAKG1-3.

28. A bifunctional chimeric monoclonal antibody wherein one light chain variable region and one corresponding heavy chain variable region comprise light and heavy chain variable regions which specifically recognize a first antigen and the second light chain variable region and second corresponding heavy chain variable region comprise light and heavy chain variable regions which specifically recognize a second and different antigen and wherein the constant regions of all light and heavy chains comprise human antibody constant regions.

29. The bifunctional chimeric monoclonal antibody of Claim 28 wherein one light chain variable region and one corresponding heavy chain variable region comprise light and heavy chain variable regions which specifically recognize human carcinoembryonic antigen and the second light chain variable region and second corresponding heavy chain variable region comprise light and heavy chain variable regions which specifically recognize metal chelates and wherein the constant regions of all light and heavy chains comprise human antibody constant regions.

30. The bifunctional chimeric monoclonal antibody of Claim 29 wherein the light chain variable region which recognize human carcinoembryonic antigen comprises the amino acid sequence:

Asp - Ile - Val - Met - Thr - Gln - Ser - Gln - Lys Phe - Met - Ser - Thr - Ser - Val - Gly - Asp - Arg Val - Ser - Ile - Thr - Cys - Lys - Ala - Ser - Gln Asn - Val - Arg - Thr - Ala - Val - Ala - Trp - Tyr Gln - Gln - Lys - Pro - Gly - Gln - Ser - Pro - Lys Ala - Leu - Ile - Tyr - Leu - Ala - Ser - Asn - Arg Tyr - Thr - Gly - Val - Pro - Asp - Arg - Phe - Thr Gly - Ser - Gly - Ser - Gly - Thr - Asp - Phe - Thr Leu - Thr - Ile - Thr - Asn - Val - Gln - Ser - Glu Asp - Leu - Ala - Asp - Tyr - Phe - Cys - Leu - Gln His - Trp - Asn - Tyr - Pro - Leu - Thr - Phe - Gly Ala - Gly - Thr - Lys - Leu - Glu - Leu - Lys - Arg

and heavy chain variable region which recognizes human carcinoembryonic antigen comprises the amino acid sequence:

Asp - Val - Gln - Leu - Val - Glu - Ser - Gly - Gly Gly - Leu - Val - Gln - Pro - Gly - Gly - Ser - Arg Lys - Leu - Ser - Cys - Ala - Ala - Ser - Gly - Phe Thr - Phe - Ser - Asn - Phe - Gly - Met - His - Trp Ile - Arg - Gln - Ala - Pro - Glu - Lys - Gly - Leu Glu - Trp - Val - Ala - Tyr - Ile - Ser - Gly - Gly Ser - Ser - Thr - Ile - Tyr - Tyr - Ala - Asp - Thr Val - Lys - Gly - Arg - Phe - Thr - Ile - Ser - Arg Asp - Asn - Pro - Arg - Asn - Thr - Leu - Phe - Leu Gln - Met - Thr - Ser - Leu - Arg - Ser - Glu - Asp Thr - Ala - Met - Phe - Tyr - Cys - Ala - Arg - Asp Tyr - Tyr - Ala - Asn - Asn - Tyr - Trp - Tyr - Phe Asp - Val - Trp - Gly - Ala - Gly - Thr - Thr - Val Thr - Val - Ser - Ser - Ala

the light chain variable region which recognizes metal chelates comprises the amino acid sequence:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

and the heavy chain variable region which recognizes metal chelates comprises the amino acid sequence:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala.

31. The bifunctional chimeric monoclonal antibody of Claim 30 that is bifunctional chimeric CEM/CHA.

Claims for the following Contracting State: GR

1. A recombinant DNA compound comprising a first DNA sequence which encodes for the human carcinoembryonic antigen-specific light chain variable region of a chimeric monoclonal antibody and a second DNA sequence which encodes for the human carcinoembryonic antigen-specific heavy chain variable region of a chimeric monoclonal antibody, wherein the antibody light chain variable region amino acid sequence comprises:

Asp - Ile - Val - Met - Thr - Gln - Ser - Gln - Lys Phe - Met - Ser - Thr - Ser - Val - Gly - Asp - Arg Val - Ser - Ile - Thr - Cys - Lys - Ala - Ser - Gln Asn - Val - Arg - Thr - Ala - Val - Ala - Trp - Tyr Gln - Gln - Lys - Pro - Gly - Gln - Ser - Pro - Lys Ala - Leu - Ile - Tyr - Leu - Ala - Ser - Asn - Arg Tyr - Thr - Gly - Val - Pro - Asp - Arg - Phe - Thr Gly - Ser - Gly - Ser - Gly - Thr - Asp - Phe - Thr Leu - Thr - Ile - Thr - Asn - Val - Gln - Ser - Glu Asp - Leu - Ala - Asp - Tyr - Phe - Cys - Leu - Gln His - Trp - Asn - Tyr - Pro - Leu - Thr - Phe - Gly Ala - Gly - Thr - Lys - Leu - Glu - Leu - Lys - Arg

and wherein the antibody heavy chain variable region amino acid sequence comprises:

Asp - Val - Gln - Leu - Val - Glu - Ser - Gly - Gly Gly - Leu - Val - Gln - Pro - Gly - Gly - Ser - Arg Lys - Leu - Ser - Cys - Ala - Ala - Ser - Gly - Phe Thr - Phe - Ser - Asn - Phe - Gly - Met - His - Trp Ile - Arg - Gln - Ala - Pro - Glu - Lys - Gly - Leu Glu - Trp - Val - Ala - Tyr - Ile - Ser - Gly - Gly Ser - Ser - Thr - Ile - Tyr - Tyr - Ala - Asp - Thr Val - Lys - Gly - Arg - Phe - Thr - Ile - Ser - Arg Asp - Asn - Pro - Arg - Asn - Thr - Leu - Phe - Leu Gln - Met - Thr - Ser - Leu - Arg - Ser - Glu - Asp Thr - Ala - Met - Phe - Tyr - Cys - Ala - Arg - Asp Tyr - Tyr - Ala - Asn - Asn - Tyr - Trp - Tyr - Phe Asp - Val - Trp - Gly - Ala - Gly - Thr - Thr - Val Thr - Val - Ser - Ser - Ala.

2. The recombinant DNA compound according to Claim 1 wherein the first DNA strand coding sequence comprises:

GAC - ATT - GTG - ATG - ACC - CAG - TCT - CAA - AAA TTC - ATG - TCC - ACA - TCA - GTA - GGA - GAC - AGG GTC - AGC - ATC - ACC - TGC - AAG - GCC - AGT - CAG AAT - GTT - CGT - ACT - GCT - GTT - GCC - TGG - TAT CAA - CAG - AAA - CCA - GGG - CAG - TCT - CCT - AAA GCA - CTG - ATT - TAC - TTG - GCA - TCC - AAC - CGG TAC - ACT - GGA - GTC - CCT - GAT - CGC - TTC - ACA GGC - AGT - GGA - TCT - GGG - ACA - GAT - TTC - ACT CTC - ACC - ATT - ACC - AAT - GTG - CAA - TCT - GAA GAC - CTG - GCA - GAT - TAT - TTC - TGT - CTG - CAA CAT - TGG - AAT - TAT - CCG - CTC - ACG - TTC - GGT GCT - GGG - ACC - AAG - CTG - GAG - CTG - AAA - CGG

and wherein the second DNA strand coding sequence comprises:

GAT - GTG - CAG - CTG - GTG - GAG - TCT - GGG - GGA GGC - TTA - GTG - CAG - CCT - GGA - GGG - TCC - CGG AAA - CTC - TCC - TGT - GCA - GCC - TCT - GGA - TTC ACT - TTC - AGT - AAC - TTT - GGA - ATG - CAC - TGG ATT - CGT - CAG - GCT - CCA - GAG - AAG - GGA - CTG GAG - TGG - GTC - GCA - TAC - ATT - AGT - GGT - GGC AGT - AGT - ACC - ATC - TAC - TAT - GCA - GAC - ACA GTG - AAG - GGC - CGA - TTC - ACC - ATC - TCC - AGA GAC - AAT - CCC - AGG - AAC - ACC - CTC - TTC - CTG CAA - ATG - ACC - AGT - CTA - AGG - TCT - GAG - GAC ACG - GCC - ATG - TTT - TAC - TGT - GCA - AGA - GAT TAC - TAC - GCT - AAC - AAC - TAC - TGG - TAC - TTC GAT - GTC - TGG - GGC - GCA - GGG - ACC - ACG - GTC ACC - GTC - TCC - TCA - GCC.

3. The recombinant DNA compound according to Claim 1 or 2 wherein the DNA construct further comprises a third DNA strand sequence which codes for the light chain constant region of the chimeric monoclonal antibody and a fourth DNA strand sequence which codes for the heavy chain constant region of the chimeric monoclonal antibody.

4. The recombinant DNA compound according to any of Claims 1, 2 or 3 wherein the first and second DNA strand coding sequences are derived from a murine hybridoma.

5. The recombinant DNA compound according to Claim 4 wherein the murine hybridoma is CEM 231.6.7.

6. The recombinant DNA compound according to Claim 3 wherein the third and fourth DNA strand coding sequences are derived from a human lymphocyte.

7. The recombinant DNA compound of Claim 6 that is plasmid pNCEMKG1 as shown in Figure 5.

8. The recombinant DNA compound of Claim 6 that is plasmid pNCEMKG1(E-) as described in Example 6F.

9. A recombinant DNA compound comprising a first DNA sequence which encodes for the chelate-specific light chain variable region of a chimeric monoclonal antibody and a second DNA sequence which encodes for the chelate-specific heavy chain variable region of a chimeric monoclonal antibody, wherein the antibody light chain variable region amino acid sequences comprises:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

and wherein the antibody heavy chain variable region amino acid sequence comprises:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala.

10. The recombinant DNA compound according to Claim 9 wherein the first DNA strand coding sequence comprises:

CAG GCT GTT GTG ACT CAG GAA TCT GCA CTC ACC ACA TCA CCT GGT GAA ACA GTC ACA CTC ACT TGT CGC TCA AGT ACT GGG GCT GTT ACA ACT AGT AAC TAT GCC AAC TGG GTC CAA GAA AAA CCA GAT CAT TTA TTC ACT GGT CTA ATA GGT GGT ACC AAT AAC CGG GCT CCA GGT GTT CCT GCC AGA TTC TCA GGC TCC CTG ATT GGA GAC AAG GCT GCC CTC ACC ATC ACA GGG GCA CAG ACT GAA GAT GAG GCA AGA TAT TTC TGT GCT CTA TGG TAC ACC AGC CTC TGG GTA TTC GGT GGA GGA ACC AAA CTG ACT GTC CTA GGG

and wherein the second DNA strand coding sequence comprises:

GAA GTG ACG CTG GTG GAG TCT GGG GGA GAC TCA GTG AAG CCT GGA GGG TCC CTG AAA CTC TCC TGT GCA GCC TCT GGA TTC ACT TTA AGT GGT GAA ACC ATG TCT TGG GTT CGC CAG ACT CCG GAG AAG AGG CTG GAG TGG GTC GCA ACC ACT CTT AGT GGT GGT GGT TTC ACC TTC TAT TCA GCC AGT GTG AAG GGT CGT TTC ACC ATC TCC AGA GAC AAT GCC CAG AAC AAC CTC TAT CTA CAA CTG AAT AGT CTG AGG TCT GAG GAC ACG GCC TTG TAT TTC TGT GCA AGT CAT CGG TTT GTT CAC TGG GGC CAC GGG ACT CTG GTC ACT GTC TCT GCA GCC.

11. The recombinant DNA compound according to Claim 1 or 2 wherein the DNA construct further comprise a third DNA strand sequence which codes for the light chain constant region of the chimeric monoclonal antibody and a fourth DNA strand sequence which codes for the heavy chain constant region of the chimeric monoclonal antibody.

12. The recombinant DNA compound according to any of Claims 9, 10 or 11 wherein the first and second DNA strand coding sequences are derived from a murine hybridoma.

13. The recombinant DNA compound according to Claim 12 wherein the murine hybridoma is CHA 255.5.

14. The recombinant DNA compound according to Claim 11 wherein the third and fourth DNA strand coding sequences are derived from a human lymphocyte.

15. The recombinant DNA compound of Claim 14 that is plasmid pGCHAKG1 as shown in Figure 10.

16. The recombinant DNA compound of Claim 14 that is plasmid pGCHAKG1(E-) as described in Example 6G.

17. The recombinant DNA compound of Claim 14 that is plasmid pGCHAKG1-2 as described in example 7B.

18. The recombinant DNA compound of Claim 14 that is plasmid pGCHAKG1-3 as described in Example 7B.

19. A transformed host cell which secretes bifunctional chimeric CEM/CHA.

20. The host cell of Claim 19 that is SP2/0/pNCEMKG1/pGCHAKG1.

21. The host cell of Claim 19 that is SP2/0/pNCEMKG1/pGCHAKG1(E-).

22. The host cell of Claim 19 that is SP2/0/pNCEMKG1/pGCHAKG1-2.

23. The host cell of Claim 19 that is SP2/0/pNCEMKG1/pGCHAKG1-3.

24. The host cell of Claim 19 that is SP2/0/pNCEMKG1(E-)/pGCHAKG1.

25. The host cell of Claim 19 that is SP2/0/pNCEMKG1(E-)/pGCHAKG1(E-).

26. The host cell of Claim 19 that is SP2/0/pNCEMKG1(E-)/pGCHAKG1-2.

27. The host cell of Claim 19 that is SP2/0/pNCEMKG1(E-)/pGCHAKG1-3.

Claims for the following Contracting State: ES

1. A method of expressing bifunctional chimeric antibody chains in transfected cells, said antibody chains comprising variable regions which bind to human carcenoembryonic antigen, said method comprising the steps of:

a) constructing a recombinant DNA vector which comprises a first gene encoding a light chain variable region with the amino acid sequence:

Asp - Ile - Val - Met - Thr - Gln - Ser - Gln - Lys Phe - Met - Ser - Thr - Ser - Val - Gly - Asp - Arg Val - Ser - Ile - Thr - Cys - Lys - Ala - Ser - Gln Asn - Val - Arg - Thr - Ala - Val - Ala - Trp - Tyr Gln - Gln - Lys - Pro - Gly - Gln - Ser - Pro - Lys Ala - Leu - Ile - Tyr - Leu - Ala - Ser - Asn - Arg Tyr - Thr - Gly - Val - Pro - Asp - Arg - Phe - Thr Gly - Ser - Gly - Ser - Gly - Thr - Asp - Phe - Thr Leu - Thr - Ile - Thr - Asn - Val - Gln - Ser - Glu Asp - Leu - Ala - Asp - Tyr - Phe - Cys - Leu - Gln His - Trp - Asn - Tyr - Pro - Leu - Thr - Phe - Gly Ala - Gly - Thr - Lys - Leu - Glu - Leu - Lys - Arg

and which further comprises a second gene encoding a heavy chain variable region with the amino acid sequence:

Asp - Val - Gln - Leu - Val - Glu - Ser - Gly - Gly Gly - Leu - Val - Gln - Pro - Gly - Gly - Ser - Arg Lys - Leu - Ser - Cys - Ala - Ala - Ser - Gly - Phe Thr - Phe - Ser - Asn - Phe - Gly - Met - His - Trp Ile - Arg - Gln - Ala - Pro - Glu - Lys - Gly - Leu Glu - Trp - Val - Ala - Tyr - Ile - Ser - Gly - Gly Ser - Ser - Thr - Ile - Tyr - Tyr - Ala - Asp - Thr Val - Lys - Gly - Arg - Phe - Thr - Ile - Ser - Arg Asp - Asn - Pro - Arg - Asn - Thr - Leu - Phe - Leu Gln - Met - Thr - Ser - Leu - Arg - Ser - Glu - Asp Thr - Ala - Met - Phe - Tyr - Cys - Ala - Arg - Asp Tyr - Tyr - Ala - Asn - Asn - Tyr - Trp - Tyr - Phe Asp - Val - Trp - Gly - Ala - Gly - Thr - Thr - Val Thr - Val - Ser - Ser - Ala

b) transfecting said vector into a mammalian host cell, and

c) culturing said host cell under conditions suitable for the expression of said variable region gene.

2. The method of Claim 1 wherein the recombinant DNA vector is plasmid pNCEMKG1 as shown in Figure 5.

3. The method of Claim 1 wherein the recombinant DNA vector is plasmid pNCEMKG1(E-) as described in Example 6F.

4. The method of Claim 2 or 3 wherein the transfected cell is an SP2/0 cell.

5. A method of expressing bifunctional chimeric antibody chains in transfected cells, said antibody chains comprising variable regions which bind to metal chelates, said method comprising the steps of:

a) constructing a recombinant DNA vector which comprises a first gene encoding a heavy chain variable region with the amino acid sequence:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

and which further comprises a second gene encoding a heavy chain variable region with the amino acid sequence:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala

    b) transfecting said vector into a mammalian host cell, and
    c) culturing said host cell under conditions suitable for the expression of said variable region.

6. The method of Claim 5 wherein the recombinant DNA vector is plasmid pGCHAKG1 as shown in Figure 10.

7. The method of Claim 5 wherein the recombinant DNA vector is plasmid pGCHAKG1(E-) as described in Example 6G.

8. The method of Claim 5 wherein the recombinant DNA vector is plasmid pGCHAKG1-2 as described in Example 7B.

9. The method of Claim 5 wherein the recombinant DNA vector is plasmid pGCHAKG1-3 as described in Example 7B.

10. The method of any one of Claims 6 through 9 wherein the transfected cell is an SP2/0 cell.

11. A method of expressing bifunctional chimeric antibodies in transfected cells, said antibodies comprising light and heavy chain variable regions which bind to human carcinoembryonic antigen and light and heavy chain variable regions which bind to metal chelates, said method comprising the steps of:

    a) constructing a recombinant DNA vector encoding a light chain variable region with the amino acid sequence:

Asp - Ile - Val - Met - Thr - Gln - Ser - Gln - Lys Phe - Met - Ser - Thr - Ser - Val - Gly - Asp - Arg Val - Ser - Ile - Thr - Cys - Lys - Ala - Ser - Gln Asn - Val - Arg - Thr - Ala - Val - Ala - Trp - Tyr Gln - Gln - Lys - Pro - Gly - Gln - Ser - Pro - Lys Ala - Leu - Ile - Tyr - Leu - Ala - Ser - Asn - Arg Tyr - Thr - Gly - Val - Pro - Asp - Arg - Phe - Thr Gly - Ser - Gly - Ser - Gly - Thr - Asp - Phe - Thr Leu - Thr - Ile - Thr - Asn - Val - Gln - Ser - Glu Asp Leu - Ala - Asp - Tyr - Phe - Cys - Leu - Gln His - Trp - Asn - Tyr - Pro - Leu - Thr - Phe - Gly Ala - Gly - Thr - Lys - Leu - Glu - Leu - Lys - Arg

and a heavy chain variable region with the amino acid sequence:

Asp - Val - Gln - Leu - Val - Glu - Ser - Gly - Gly Gly - Leu - Val - Gln - Pro - Gly - Gly - Ser - Arg Lys - Leu - Ser - Cys - Ala - Ala - Ser - Gly - Phe Thr - Phe - Ser - Asn - Phe - Gly - Met - His - Trp Ile - Arg - Gln - Ala - Pro - Glu - Lys - Gly - Leu Glu - Trp - Val - Ala - Tyr - Ile - Ser - Gly - Gly Ser - Ser - Thr - Ile - Tyr - Tyr - Ala - Asp - Thr Val - Lys - Gly - Arg - Phe - Thr - Ile - Ser - Arg Asp - Asn - Pro - Arg - Asn - Thr - Leu - Phe - Leu Gln - Met - Thr - Ser - Leu - Arg - Ser - Glu - Asp Thr - Ala - Met - Phe - Tyr - Cys - Ala - Arg - Asp Tyr - Tyr - Ala - Asn - Asn - Tyr - Trp - Tyr - Phe Asp - Val - Trp - Gly - Ala - Gly - Thr - Thr - Val Thr - Val - Ser - Ser - Ala

and

    b) constructing a recombinant DNA vector encoding a light chain variable region with the amino acid sequence:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Gly Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

and a heavy chain variable region with the amino acid sequence:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala

or

    c) constructing a recombinant DNA vector which comprises genes encoding human carcinoembryonic antigen-specific light and heavy chain variable regions and genes encoding chelate specific light and heavy chain variable regions and

d) transfecting said vectors a) and b) or said vector c) into a mammalian host cell, and

e) culturing said host cell under conditions suitable for the expression of said variable region genes.

12. The method of Claim 11 wherein the plasmid of a) is plasmid pNCEMKG1 and the plasmid of b) is plasmid pGCHAKG1.

13. The method of Claim 11 wherein the plasmid of a) is plasmid pNCEMKG1 and the plasmid of b) is plasmid pGCHAKG1(E-).

14. The method of Claim 11 wherein the plasmid of a) is plasmid pNCEMKG1 and the plasmid of b) is plasmid pGCHAKG1-2.

15. The method of Claim 11 wherein the plasmid of a) is plasmid pNCEMKG1 and the plasmid of b) is plasmid pGCHAKG1-3.

16. The method of Claim 11 wherein the plasmid of a) is plasmid pNCEMKG1(E-) and the plasmid of b) is plasmid pGCHAKG1.

17. The method of Claim 11 wherein the plasmid of a) is plasmid pNCEMKG1(E-) and the plasmid of b) is plasmid pGCHAKG1(E-).

18. The method of Claim 11 wherein the plasmid of a) is plasmid pNCEMKG1(E-) and the plasmid of b) is plasmid pGCHAKG1-2.

19. The method of Claim 11 wherein the plasmid of a) is plasmid pNCEMKG1(E-) and the plasmid of b) is plasmid pGCHAKG1-3.

20. The method of any one of Claims 12 through 19 wherein the transfected cell is an SP2/0 cell.

## FIG.I
## Restriction Site and Function Maps of Plasmids
## pMLCE-10 and pHKF-1

pMLCE-10

pHKF-1

# FIG.2
# Restriction Site and Function Maps of Plasmids pHKCe-10 and pGCEMK

pHKCe-10

pGCEMK

Neu eingereicht
Nouvellement

# FIG.3
# Restriction Site and Function Maps of Plasmids
# pMHCE-30 and pHG1Z

pMHCE-30

pHG1Z

## FIG. 4
## Restriction Site and Function Maps of Plasmids pHGCEM-30 and pNCEMG1

# FIG. 5
# Restriction Site and Function Map of Plasmid pNCEMKG1

# FIG.6
# Restriction Site and Function Map of Plasmid pMLCH1

Eco RI

Hind III

Bam HI

Bam HI (this Bam HI deleted in pMLCH1dB)

Bst XI

Bst XI

Bst XI

Hind III

(16,6 kb)

pSV2neo

Sst I

Pst I

Eco RI

Cha V Lambda

Bam HI

Sst I

CHA C Lambda

# FIG.7
## Restriction Site and Function Map of Plasmid pGCHAK

# FIG.8
## restriction Site and Function Map of Plasmids pUCVHInc-1A and pHG1-CHA

pUCVHInc-1A

pHG1-CHA

# FIG.9
## Restriction Site and Function Map of Plasmid pGCHAG1

# FIG. 10
## Restriction Site and Function Map of Plasmid pGCHAKG1